Europäisches Patentamt

European Patent Office

Office européen des brevets

(19)

(11) Publication number: **0 238 323 B1**

# EUROPEAN PATENT SPECIFICATION

(45) Date of publication of patent specification: **28.12.94**

(21) Application number: **87302318.8**

(22) Date of filing: **18.03.87**

(51) Int. Cl.5: **C12N  15/00**, C12N 9/00, C12N 1/20, C12P 19/62, //(C12N1/20,C12R1:19,1:465)

(54) **Improvements in or relating to tylosin antibiotic-producing microorganisms.**

(30) Priority: **21.03.86 US 842330**
**25.07.86 US 890670**

(43) Date of publication of application:
**23.09.87 Bulletin  87/39**

(45) Publication of the grant of the patent:
**28.12.94 Bulletin  94/52**

(84) Designated Contracting States:
**AT BE CH DE ES FR GB GR IT LI LU NL SE**

(56) References cited:

**ANTIMICROBIAL AGENTS AND CHEMO-THERAPY, vol. 21, no. 5, May 1982, pages 758-763, Washington, DC, US; E.T. SENO et al.: "S-adenosyl-L-methionine: macrocin O-methyltransferase activities in a series of Streptomyces fradiae mutants that produce different levels of the macrolide antibiotic tylosin"**

(73) Proprietor: **ELI LILLY AND COMPANY**
**Lilly Corporate Center**
**Indianapolis**
**Indiana 46285 (US)**

(72) Inventor: **Cox, Karen Leigh**
**Box 204 Painted Hills**
**Martinsville**
**Indiana 46151 (US)**
Inventor: **Fishman, Scott Eric**
**5871 Hillside Avenue**
**Indianapolis**
**Indiana 46220 (US)**
Inventor: **Hershberger, Charles Lee**
**Rural Route No. 1, Box 343**
**New Palestine**
**Indiana 46163 (US)**
Inventor: **Seno, Eugene Thomas**
**5109 East 72nd Street**
**Indianapolis**
**Indiana 46250 (US)**

EP 0 238 323 B1

ABSTRACTS OF THE ANNUAL MEETING OF THE AMERICAN SOCIETY FOR MICROBIOLO-GY, vol.83, 1983, page 110, no. H25, Washington, DC, US; S.E. FISHMAN et al.:"Construction and characterization of a genomic library of Streptomyces fradiae"

JOURNAL OF BACTERIOLOGY, vol. 163, no. 1, July 1985, pages 180-185, American Society for Microbiology, Washington, DC, US; P. MATSUSHIMA et al.: "Efficient plasmid transformation of Streptomyces ambofaciens and Streptomyces fradiae protoplasts"

ABSTRACTS OF THE ANNUAL MEETING OF THE AMERICAN SOCIETY FOR MICROBIOLO-GY, vol.85, 1985, page 195, no. K 141, Washington, DC, US; N.J. BAUER et al.:"Purification and properties of S-adenosyl-L-methionine: Macrocin O-methyltransferase from Streptomyces fradiae"

JOURNAL OF NATURAL PRODUCTS, vol. 49, no. 6, November/December 1986, pages 971-980; K.L. COX et al.: "The use of recombinant DNA techniques to study tylosin biosynthesis and resistance in Streptomyces fradiae"

ANTIMICROBIAL AGENTS AND CHEMO-THERAPY, vol. 20, no. 2, August 1981, pages 214-225, Washington, DC, US; R.H. BALTZ et al.: "Properties of Streptomyces fradiae mutants blocked in biosynthesis of the macrolide antibiotic tylosin"

PEPTIDE SCIENCE, vol. 16, no. 4, 1985, pages 425-426, GB; D.A. HOPWOOD:"Genetics and the production of new molecules"

(74) Representative: Hudson, Christopher Mark et al
Lilly Industries Limited
European Patent Operations
Erl Wood Manor
Windlesham
Surrey GU20 6PH (GB)

**Description**

The present invention provides a novel method for increasing the antibiotic-producing ability of an antibiotic-producing organism. The method involves transforming a microbial host cell with a DNA sequence that codes for the expression of a gene product that is rate-limiting in the desired antibiotic's biosynthetic pathway. The invention also provides related DNA sequences that code for antibiotic biosynthetic gene products, recombinant DNA expression vectors, and transformed microbial host cells.

The present invention represents an early and significant commercial exploitation of recombinant DNA technology in antibiotic-producing organisms such as streptomycetes. Prior to the present invention, the development and exploitation of recombinant DNA technology has been limited, for the most part, to the expression of specific polypeptides in E. coli and, in some instances, mammalian cells. These advances led to the comparatively simple expression of heterologous gene products such as human insulin A and B chains, human proinsulin, human growth hormone, human protein C, human tissue plasminogen activator, bovine growth hormone, and several other compounds of potential value. In each case, heterologous gene expression is more or less independent and does not interact with, take part in, or modulate operative biosynthetic pathways. Recombinant DNA technology can now be applied to improve selected biosynthetic pathways for the expression of increased yields of antibiotics or antimicrobial precursors.

Most recombinant DNA technology applied to streptomycetes and other antibiotic-producing organisms has been limited to the development of cloning vectors. Early attempts include the disclosures of Reusser U.S. Patent No. 4,332,898 and Manis et al. U.S. Patent Nos. 4,273,875; 4,332,900; 4,338,400; and 4,340,674. Transformation of streptomycetes was not disclosed or taught in these early references. Improved vectors showing greater potential for use in antibiotic-producing organisms were disclosed, for example, by Fayerman et al. in U.S. Patent No. 4,513,086; and Nakatsukasa et al. in U.S. Patent Nos. 4,513,085 and 4,416,994. These improved vectors contain markers that are selectable in streptomycetes, can be used to transform many important Streptomyces strains, and constitute the tools required for conducting more complicated gene cloning experiments.

One such experiment was recently reported by Hopwood et al., 1985, in Nature 314:642. Although Hopwood et al. reported the production of novel hybrid antibiotic pigments, the disclosure does not focus on increasing the antibiotic-producing ability or biosynthetic efficiency of a given host cell but instead describes the transferring of actinorhodin pigment biosynthetic genes from one Streptomyces strain to another.

The present invention is particularly useful because it allows commercial application of recombinant DNA technology to streptomycetes and other antibiotic-producing organisms. Because over half of the clinically important antibiotics are produced by streptomycetes, it is especially desirable to develop methods that are applicable to these organisms. The present invention provides such methods and allows for the cloning of genes both for increasing the antibiotic-producing ability as well as for the production of new antibiotics and antibiotic precursors in an antibiotic-producing organism.

For purposes of the present invention, the following terms are as defined:

Antibiotic - a substance produced by a microorganism that, either naturally or with limited chemical modification, inhibits or prevents the growth of or kills another microorganism or eukaryotic cell.

Antibiotic Biosynthetic Gene - a DNA segment that encodes an enzymatic activity or encodes a product that regulates expression of an enzymatic activity and which is necessary for an enzymatic reaction for converting primary metabolites to antibiotic intermediates, which also may possess antibiotic activity, and perhaps then to antibiotics.

Antibiotic Biosynthetic Pathway - the entire set of antibiotic biosynthetic genes and biochemical reactions necessary for the process of converting primary metabolites to antibiotic intermediates and then to antibiotics.

Antibiotic-Producing Microorganism - any organism, including, but not limited to Actinoplanes, Actinomadura, Bacillus, Cephalosporium, Micromonospora, Penicillium, Nocardia, and Streptomyces, that either produces an antibiotic or contains genes that, if expressed, would produce an antibiotic.

Antibiotic Resistance-Conferring Gene - a DNA segment that encodes an activity that confers resistance to an antibiotic.

ApR - the ampicillin-resistant phenotype or gene conferring same.

Host Cell - an organism, including a viable protoplast thereof, which can be transformed with a recombinant DNA cloning vector.

NmR - the neomycin-resistant phenotype or gene conferring same.

Operation of Antibiotic Biosynthetic Pathway - the expression of antibiotic biosynthetic genes and the related biochemical reactions required for the conversion of primary metabolites into antibiotics.

Recombinant DNA Cloning Vector - any selectable and autonomously replicating or chromosomally integrating agent, including but not limited to plasmids and phages, comprising a DNA molecule to which additional DNA can be or has been added.

rep - as used in the Figures, a Streptomyces plasmid origin of replication.

Restriction Fragment - any linear DNA generated by the action of one or more restriction enzymes.

Sensitive Host Cell - a host cell, including a viable protoplast thereof, which cannot grow in the presence of a given antibiotic without a DNA segment that confers resistance thereto.

Transformant - a recipient host cell, including a viable protoplast thereof, which has undergone transformation.

Transformation - the introduction of DNA into a recipient host cell, including a viable protoplast thereof, that changes the genotype of the recipient cell.

tsr - the thiostrepton-resistant phenotype or gene conferring same.

The plasmid and chromosomal maps depicted in the Figures are drawn approximately to scale. However, the tylosin biosynthetic genes, although linked, are scattered across a large segment of DNA. Therefore, detailed restriction site mapping data exists only for small regions of the large tylosin biosynthetic gene-containing DNA fragment. The maps do not necessarily provide an exhaustive listing of all the cut sites of a given restriction enzyme. The location of individual genes, represented by line segments on the maps, was determined by deletion mapping and thus only approximates the exact location of a given gene.

Figure 1 - The Tylosin Biosynthetic Pathway.

Figure 2 - Restriction Site and Function Map of Plasmid pHJL280.

Figure 3 - Restriction Site and Function Map of Plasmid pHJL284.

Figure 4 - Restriction Site and Function Map of Plasmid pHJL309.

Figure 5 - Restriction Site and Function Map of Plasmid pHJL311.

Figure 6 - Restriction Site and Function Map of Plasmid pHJL315.

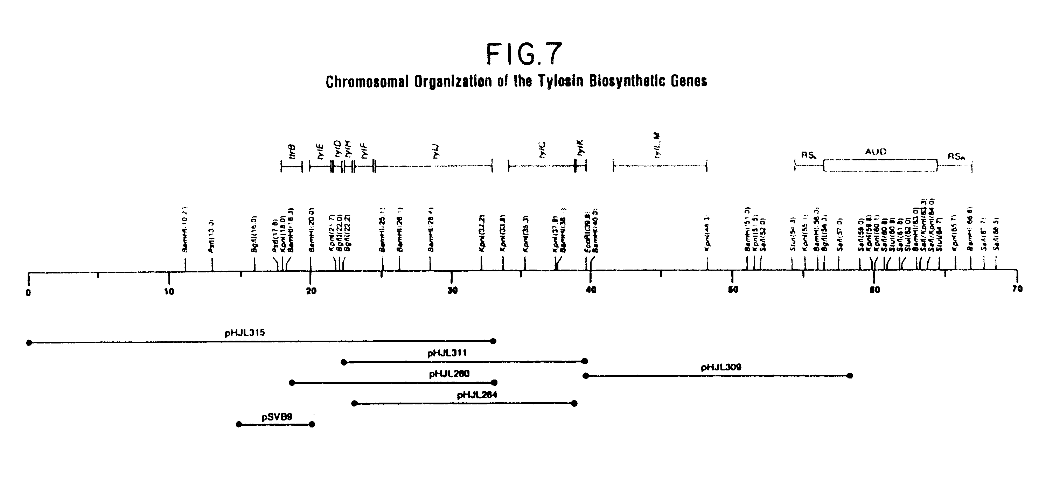

Figure 7 - Chromosomal Organization of the Tylosin Biosynthetic Genes.

The present invention provides a method for increasing the antibiotic- or antibiotic precursor-producing ability of an antibiotic-producing microorganism, which comprises culturing a microorganism which produces an antibiotic or antibiotic precursor by a biosynthetic pathway, said microorganism being transformed with a DNA cloning vector or portion thereof which contains an antibiotic or antibiotic-precursor biosynthetic gene coding for expression of a rate-limiting enzyme or gene product of the biosynthetic pathway, under conditions suitable for cell growth, expression of the antibiotic or antibiotic-precursor biosynthetic gene and production of the antibiotic or antibiotic precursor, provided that the culturing process provides an increase in the antibiotic-producing ability of the microorganism.

The invention further provides related antibiotic biosynthetic genes, recombinant DNA cloning vectors, and antibiotic or antibiotic precursor-producing microorganisms transformed with the genes and vectors.

Further, there is provided a process for preparing an antibiotic, an antibiotic precursor, or a pharmaceutically acceptable salt thereof, which comprises culturing a microorganism which produces an antibiotic or antibiotic precursor through an antibiotic biosynthetic pathway, said microorganism being transformed with a DNA cloning vector, or portion thereof, in a culture medium containing assimilable sources of carbon, nitrogen and inorganic salts under aerobic fermentation conditions characterized in that the DNA cloning vector, or portion thereof, comprises an antibiotic biosynthetic gene which codes for the expression of a rate-limiting enzyme or gene product of the antibiotic biosynthetic pathway, said antibiotic biosynthetic gene being expressed under fermentation conditions providing for an increase in the antibiotic-producing ability of the microorganism.

The methods of the present invention are widely applicable to all antibiotic-producing organisms. The following tables provide a non-exhaustive list of antibiotic producing organisms to which the present invention may apply.

4

EP 0 238 323 B1

## TABLE I

Aminocyclitol Antibiotic-Producing Organisms

| Organism | Antibiotic |
|---|---|
| **Bacillus** | |
| various species | various aminocyclitols |
| **Micromonospora** | |
| various species | gentamycins |
| **Saccharopolyspora** | |
| various species | various aminocyclitols |
| **Streptomyces** | |
| albogriseolus | neomycins |
| albus var. metamycinus | metamycin |
| aquacanus | N-methyl hygromycin B |
| atrofaciens | hygromycins |
| bikiniensis | streptomycin |
| bluensis var. bluensis | bluensomycin |
| canus | ribosyl paromamine |
| catenulae | catenulin |
| chrestomyceticus | aminosidine |
| crystallinus | hygromycin A |
| erythrochromogenes var. narutoensis | streptomycin |
| eurocidicus | A16316-C |
| fradiae | hybrimycins and neomycins |
| fradiae var. italicus | aminosidine |

5

TABLE I (Continued)

| Organism | Antibiotic |
|---|---|
| Streptomyces | |
| galbus | streptomycin |
| griseus | streptomycin |
| griseoflavus | MA 1267 |
| hofuensis | seldomycin complex |
| hygroscopicus | hygromycins, leucanicidin, and hygrolidin |
| hygroscopicus forma glebosus | glebomycin |
| hygroscopicus var. limoneus | validamycins |
| hygroscopicus var. sagamiensis | spectinomycin |
| kanamyceticus | kanamycin A and B |
| kasugaensis | kasugamycins |
| kasugaspinus | kasugamycins |
| lavendulae | neomycin |
| lividus | lividomycins |
| mashuensis | streptomycin |
| microsporeus | SF-767 |
| netropsis | LL-AM31 |
| noboritoensis | hygromycins |
| olivaceus | streptomycin |
| olivoreticuli var. cellulophilus | destomycin A |

6

EP 0 238 323 B1

## TABLE I (Continued)

| Organism | Antibiotic |
|---|---|
| poolensis | streptomycin |
| rameus | streptomycin |
| ribosidificus | SF733 |
| rimofaciens | destomycin A |
| rimosus forma paromomycinus | paromomycins and catenulin |
| spectabilis | spectinomycin |
| tenebrarius | tobramycin and apramycin |
| Streptoverticillium flavopersicus | spectinomycin |

TABLE II

| Ansamycin Antibiotic-Producing Organisms | |
|---|---|
| Organism | Antibiotic |
| Micromonospora | |
| various species | various ansamycins |
| Nocardia | |
| mediterranei | rifamycin |
| Streptomyces | |
| collinus | ansatrienes and napthomycins |
| diastochromogenes | ansatrienes and napthomycins |
| galbus subsp. griseosporeus | napthomycin B |
| hygroscopicus | herbimycin |
| hygroscopicus var. geldanus var. nova | geldamycin |
| nigellus | 21-hydroxy-25-demethyl 25-methylthioproto streptovaricin |
| rishiriensis | mycotrienes |
| sp. E/784 | actamycin and mycotrienes |
| sp. E88 | mycotrienes |
| spectabilis | streptovaricins |
| tolypophorous | tolypomycin |

7

TABLE III

| Anthracycline and Quinone Antibiotic-Producing Organisms | |
|---|---|
| Organism | Antibiotic |
| Streptomyces | |
| caespitosus | mitomycins A, B, and C |
| coelicolor | actinorhodin |
| coeruleorubidicus | daunomycin |
| cyaneus | ditrisarubicin |
| flavogriseus | cyanocycline A |
| galilaeus | aclacinomycin A, auramycins, and sulfurmycins |
| lusitanus | napthyridinomycin |
| peuceticus | daunomycin and adriamycin |
| violochromogenes | arugomycin |
| E | |
| | MM 4550, and MM 13902 |
| cattleya | thienamycin |
| chartreusis | SF 1623 and cephamycin A and B |

EP 0 238 323 B1

## TABLE IV

β-Lactam Antibiotic-Producing Organisms

| Organism | Antibiotic |
|---|---|
| Agrobacterium | various β-lactams |
| Cephalosporium | |
| acremonium | penicillins and cephalosporins |
| Chromobacterium | various β-lactams |
| Gluconobacter | various β-lactams |
| Nocardia | |
| lactamadurans | cephamycin C |
| uniformis | nocardicin |
| Penicillium | |
| chrysogenum | various penicillins and other β-lactams |
| Serratia | various β-lactams |
| antibioticus | clavulanic acid |
| argenteolus | asparenomycin A, |

9

## TABLE IV (Continued)

| Organism | Antibiotic |
|---|---|
| Streptomyces | |
| cinnamonensis | cephamycin A and B |
| clavuligerus | PA-32413-I, cephamycin C, A16886A, penicillins cephalosporins, |
| clavulanic | |
| | acid, and other clavams |
| fimbriatus | cephamycin A and B |
| flavovirens | MM 4550 and MM 13902 |
| flavus | MM 4550 and MM 13902 |
| fulvoviridis | MM 4550 and MM 13902 |
| griseus | cephamycin A and B and carpetimycin A and B |
| halstedi | cephamycin A and B |
| heteromorphus | C2081X and cephamycin A and B |
| hygroscopicus | deacetoxycephalosporin C |
| lipmanii | cephamycin, penicillin N, 7-methoxycephalosporin C, A16884, MM4550, MM13902 |
| olivaceus | epithienamycin F, MM 4550, and MM 13902 |
| panayensis | C2081X and cephamycin A and B |
| rochei | cephamycin A and B |
| sioyaensis | MM 4550 and MM 13902 |
| sp. OA-6129 | OA-6129A |
| sp. KC-6643 | carpetimycin A |
| viridochromogenes | cephamycin A and B |
| wadayamensis | WS-3442-D |

TABLE V

Macrolide, Lincosamide, and Streptogramin
Antibiotic-Producing Organisms

| Organism | Antibiotic |
|---|---|
| Micromonospora | |
| rosaria | rosaramicin |
| Streptomyces | |
| albireticuli | carbomycin |
| albogriseolus | mikonomycin |
| albus | albomycetin |
| albus var. | |
| coilmyceticus | coleimycin |
| ambofaciens | spiramycin and |
| | foromacidin D |
| antibioticus | oleandomycin |
| avermitilis | avermectins |
| bikiniensis | chalcomycin |
| bruneogriseus | albocycline |
| caelestis | M188 and celesticetin |
| cinerochromogenes | cineromycin B |
| cirratus | cirramycin |
| deltae | deltamycins |
| djakartensis | niddamycin |
| erythreus | erythromycins |
| eurocidicus | methymycin |
| eurythermus | angolamycin |
| fasciculus | amaromycin |

## TABLE V (Continued)

| Organism | Antibiotic |
|---|---|
| Streptomyces | |
| felleus | argomycin and picromycin |
| fimbriatus | amaromycin |
| flavochromogenes | amaromycin and shincomycins |
| fradiae | tylosin |
| fungicidicus | NA-181 |
| fungicidicus var. espinomyceticus | espinomycins |
| furdicidicus | mydecamycin |
| goshikiensis | bandamycin |
| griseofaciens | PA133A and B |
| griseoflavus | acumycin |
| griseofuscus | bundlin |
| griseolus | griseomycin |
| griseospiralis | relomycin |
| griseus | borrelidin |
| griseus ssp. sulphurus | bafilomycins |
| halstedi | carbomycin and leucanicidin |
| hygroscopicus | tylosin |
| hygroscopicus subsp. aureolacrimosus | milbemycins |
| kitastoensis | leucomycin A$_3$ and josamycin |
| lavendulae | aldgamycin |
| lincolnensis | lincomycin |

12

## TABLE V (Continued)

| Organism | Antibiotic |
|---|---|
| loidensis | vernamycin A and B |
| macrosporeus | carbomycin |
| maizeus | ingramycin |
| mycarofaciens | acetyl-leukomycin, and espinomycin |
| narbonensis | josamycin and narbomycin |
| narbonensis var. josamyceticus | leucomycin $A_3$ and josamycin |
| olivochromogenes | oleandomycin |
| platensis | platenomycin |
| rimosus | tylosin and neutramycin |
| rochei | lankacidin and borrelidin |
| rochei var. volubilis | T2636 |
| roseochromogenes | albocycline |
| roseocitreus | albocycline |
| spinichromogenes var. suragaoensis | kujimycins |
| tendae | carbomycin |
| thermotolerans | carbomycin |
| venezuelae | methymycins |
| violaceoniger | lankacidins and lankamycin |

13

## TABLE VI

### Miscellaneous Antibiotic-Producing Streptomyces

| Antibiotic Type | Streptomyces Species | Antibiotic |
|---|---|---|
| amino acid analogues | sp. | cycloserine |
| cyclopentane ring-containing | coelicolor | methylenomycin A |
| | erythrochromogenes | sarkomycin |
| | kasugaensis | aureothricin and thiolutin |
| | violaceoruber | methylenomycin A |
| nitro-containing | venezuelae | chloramphenicol |
| polyenes | griseus | candicidin |
| | nodosus | amphotericin B |
| | noursei | nystatin |
| tetracyclines | aureofaciens | tetracycline, chlor tetracycline, demethyltetra cycline, and demethylchlortetra cycline |
| | rimosus | oxytetracycline |

14

TABLE VII

| Nucleoside Antibiotic-Producing Organisms | |
|---|---|
| Organism | Antibiotic |
| Corynebacterium | |
| michiganese pv. rathayi | tunicamycin analogues |
| Nocardia | |
| candidus | pyrazofurin |
| Streptomyces | |
| antibioticus<br>chartreusis<br>griseoflavus var. thuringiensis<br>griseolus<br>lysosuperificus | ara-A<br>tunicamycin<br>streptoviridans<br>sinefungin<br>tunicamycin |

TABLE VIII

| Peptide Antibiotic-Producing Organisms | |
|---|---|
| Organism | Antibiotic |
| Actinoplanes | |
| missouriensis<br>teichomyceticus | actaplanin<br>teicoplanin |
| Bacillus | |
| various species | bacitracin, polymixin, and colistin |
| Nocardia | |
| candidus<br>lurida<br>orientalis | A-35512 and avoparcin<br>ristocetin<br>vancomycin |
| Streptomyces | |
| antibioticus<br>aureus<br>canus<br>eburosporeus<br>haranomachiensis<br>pristinaespiralis<br>roseosporus<br>toyocaensis<br>virginiae | actinomycin<br>thiostrepton<br>amphomycin<br>LL-AM374<br>vancomycin<br>pristinamycin<br>lipopeptides, such as A21978C<br>A47934<br>A41030 |

15

TABLE IX

Polyether Antibiotic-Producing Organism

| Organism | Antibiotic |
|---|---|
| Actinomadura | |
| various species | various polyethers |
| oligosporus | A80190 |
| | |
| Dactylosporangium | |
| various species | various polyethers |
| | |
| Nocardia | |
| various species | various polyethers |
| | |
| Streptomyces | |
| albus | A204, A28695A and B, and salinomycin |
| aureofaciens | narasin |
| bobili | A80438 |
| cacaoi var. | |
| asoensis | lysocellin |
| chartreusis | A23187 |
| cinnamonensis | monensin |
| conglobatus | ionomycin |
| eurocidicus var. | |
| asterocidicus | laidlomycin |
| flaveolus | CP38936 |
| gallinarius | RP 30504 |
| griseus | grisorixin |

16

EP 0 238 323 B1

## TABLE IX continued

| Organism | Antibiotic |
|---|---|
| hygroscopicus | A218, emericid, DE3936, A120A, A28695A and B, etheromycin, and dianemycin |
| lasaliensis | lasalocid |
| longwoodensis | lysocellin |
| mutabilis | S-11743a |
| pactum | A80438 |
| ribosidificus | lonomycin |
| violaceoniger | nigericin |
| Streptoverticillium various species | polyethers |

The present invention is best exemplified by transforming antibiotic-producing microorganisms with genes that code for enzymes that catalyze chemical reactions governing the conversion of primary metabolites into antibiotics. One such enzyme, macrocin O-methyltransferase, catalyzes the final step in the biosynthesis of tylosin. Transforming tylosin-producing microorganisms with a macrocin O-methyltransferase-encoding gene, designated as tylF, results in an improved tylosin biosynthetic pathway because observed are increased levels of the tylF gene product in the transformed cells.

Accordingly, the present invention also provides a method for increasing the tylosin or tylosin precursor-producing ability of a tylosin-producing microorganism, which comprises culturing a microorganism which produces tylosin or a tylosin precursor by a biosynthetic pathway, said microorganism being transformed with a DNA cloning vector or portion thereof which contains tylosin or tylosin-precursor biosynthetic gene coding for expression of a rate-limiting enzyme or gene product of the biosynthetic pathway, under conditions suitable for cell growth, expression of the tylosin or tylosin-precursor biosynthetic gene and production of the tylosin or tylosin precursor, provided that the culturing process provides an increase in the tylosin- or tylosin precursor-producing ability of the microorganism.

The present invention utilizes antibiotic biosynthetic genes to increase the antibiotic-producing ability of an organism. A small number of antibiotic biosynthetic genes have been cloned, characterized, and described in the relevant literature. Methods for isolating antibiotic biosynthetic genes have been developed, but one especially preferred method is described in Baltz et al., U.S. Patent Application serial number 742,349, filed June 7, 1985, (equivalent to European Pat. Appl. No. 86304239.6, Pub. No. 204,549) which is incorporated by reference. The present tylosin antibiotic biosynthetic genes used in a specific exemplification of the present method initially were isolated from a λ library constructed in substantial accordance with the procedure described in Fishman et al., 1985, J. Bacteriology 161:199-206.

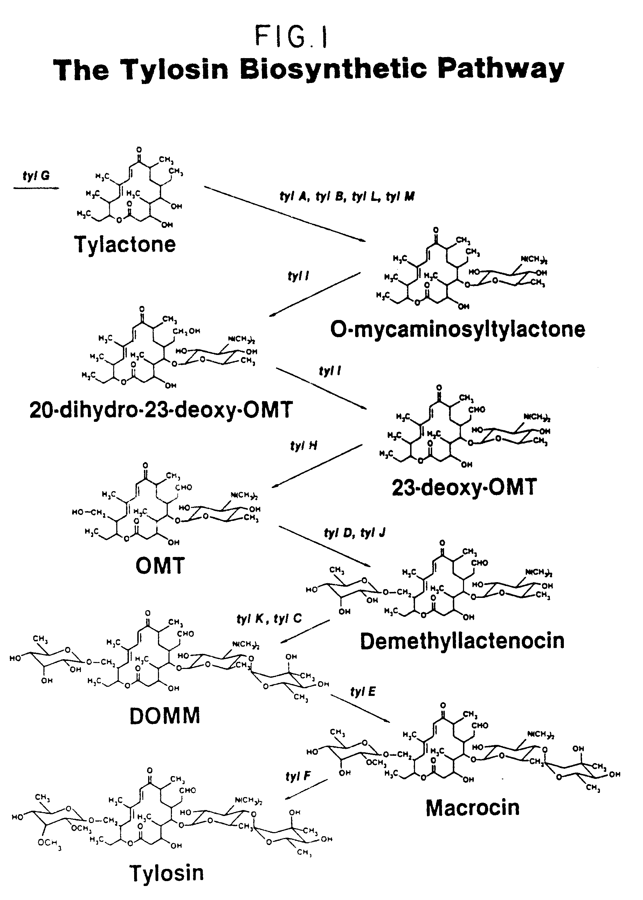

A schematic representation of the tylosin biosynthetic pathway is presented in Figure 1; each arrow in Figure 1 represents a step which is catalyzed by one or more tylosin biosynthetic gene products. The gene(s) responsible for each conversion is indicated above each arrow. Each genotypic designation may represent a class of genes that contribute to the same phenotype. A number of expression vectors are used to exemplify the present invention. These vectors comprise one or more tylosin biosynthetic genes and can be obtained from the Northern Regional Research Laboratories (NRRL), Peoria, Illinois 61604. Table X provides a brief description of each of the plasmids used to exemplify the method of the present invention.

17

Table X

Plasmids Comprising Tylosin Biosynthetic Genes

| Host/Designation | Tylosin Gene(s) | NRRL Accession No. | Date of Deposit | Map |
|---|---|---|---|---|
| E. coli K12 HB101/pHJL280 | D, E, F, H, J | B-18043 | February 18, 1986 | Fig. 2 |
| E. coli K12 HB101/pHJL284 | C, F, J | B-18044 | February 18, 1986 | Fig. 3 |
| E. coli K12 HB101/pHJL309 | L, M | B-18045 | February 18, 1986 | Fig. 4 |
| E. coli K12 HB101/pHJL311 | C, F, J, K, H | B-18046 | February 18, 1986 | Fig. 5 |
| E. coli K12 JM109/pHJL315 | D, E, F, H, J | B-18047 | February 18, 1986 | Fig. 6 |

A number of Streptomyces fradiae strains are described which have mutant tylosin biosynthetic genes and, therefore, make much less tylosin than the strain from which they were derived. Table XI provides a brief description of these mutant strains.

18

Table XI

Streptomyces fradiae Mutants Defective in Tylosin Biosynthesis

| Strain Designation | Mutant Gene | ATCC* or NRRL Accession No. |
|---|---|---|
| GS15 | tylF | NRRL 18058 (deposited March 19, 1986) |
| GS16 | tylE | ATCC 31664 (publicly available) |
| GS28 | tylF | NRRL 18059 (deposited March 19, 1986) |
| GS33 | tylL | |
| GS48 | tylD | NRRL 12170 (publicly available) |
| GS52 | tylC | NRRL 18060 (deposited March 19, 1986) |
| GS62 | tylM | |
| GS76 | tylD | |
| GS85 | tylH | NRRL 12171 (publicly available) |
| | tylK | |
| GS88 | tylJ | |

*ATCC is the American Type Culture Collection, Rockville, MD 20852, and NRRL is the Northern Regional Research Laboratory, Peoria, IL 61604.

Plasmids pHJL280, pHJL284, and pHJL315 were used to transform Streptomyces fradiae GS15 and Streptomyces fradiae GS28. The GS15 and GS28 strains were prepared from S. fradiae C4 by nitrosoguanidine mutagenesis. S. fradiae C4 was derived from S. fradiae T59235 (ATCC 19609) by mutagenesis. The GS15 strain makes almost no tylosin, and the GS28 strain makes low levels of tylosin, as compared with the C4 strain. The decreased or nonexistent tylosin-producing ability of the GS15 and GS28 strains is believed to result from mutations affecting the tylF gene, which encodes macrocin O-methyltransferase (MOMT). The MOMT enzyme, which is required for the conversion of macrocin to tylosin in the tylosin biosynthetic pathway, is frequently present in reaction rate-limiting amounts in tylosin-producing strains. Plasmids pHJL280, pHJL284, and pHJL315 remove this reaction limitation by providing a means for increasing both the copy number of the tylF biosynthetic gene and also the concentration of macrocin O-methyltransferase available for tylosin biosynthesis. Accordingly, fermentation of S. fradiae GS15/pHJL280, S. fradiae GS15/pHJL284, S. fradiae GS15/pHJL315, S. fradiae GS28/pHJL284, S. fradiae GS28/pHJL280, and S. fradiae GS28/pHJL315 for 72 hours results in about a 2-fold to a 6-fold increase in the production of macrocin O-methyltransferase over that produced in the C4 strain and a 120-fold increase over that produced in the GS28 strain.

Plasmid pHJL280 was also used to transform: (1) Streptomyces fradiae GS16; (2) S. fradiae GS48; (3) S. fradiae GS76; and (4) S. fradiae GS88 which produce tylosin below detection limits and were derived by mutagenesis of the C4 strain. Untransformed strains GS16, GS48, GS76, and GS88 respectively produce a defective enzyme or a rate-limiting amount of (1) the tylE, demethylmacrocin O-methyltransferase, enzyme; (2) the tylD enzyme, which is required for addition or biosynthesis of 6-deoxy-D-allose; (3) the tylH enzyme, which is required for oxidation of the C-23 methyl position of tylactone; and (4) the tylJ enzyme. Untransformed strains GS16, GS48, GS76, and GS88, respectively, tend to accumulate demethylmacrocin, demycinosyl tylosin, 23-deoxydemycinosyl tylosin, and demycinosyl tylosin rather than the desired tylosin antibiotic compound.

Plasmid pHJL280 provides a means for increasing the efficiency of the tylosin biosynthetic pathway by not only providing a non-defective gene but also by increasing the copy number of the tylD, tylE, tylH, and tylJ biosynthetic genes and by increasing the intracellular amount of the products specified by these genes. The concentration of available tylE gene product, therefore, is increased, resulting in an elevated amount of enzyme capable of driving the conversion of demethylmacrocin to macrocin to tylosin in the tylosin biosynthetic pathway. Similarly, the concentration of available tylD, tylH, and tylJ gene products is also increased, resulting in the production of elevated amounts of the enzymes capable of driving the 6-deoxy-D-allose addition and C-23 oxidation of tylosin precursors. Fermentation of Streptomyces fradiae GS16/pHJL280, S. fradiae GS48/pHJL280, S. fradiae GS76/pHJL280, and S. fradiae GS88/pHJL280 for 144-168 hours results in yields of tylosin that are significantly increased over that of the untransformed, low-tylosin-producing, mutant strains. Such transformed strains have higher enzyme levels of the particular enzymes encoded on plasmid pHJL280 than the parent C4 strain and thus further exemplify the present invention. Plasmid pHJL280 can be used to improve the tylosin-producing ability of any organism in which the tylD, tylE, tylF, tylH, or tylJ gene products (or any combination thereof) are present in rate-limiting amounts for tylosin biosynthesis.

Plasmid pHJL284 was also used to transform Streptomyces fradiae GS52, a low tylosin-producing, mutant strain derived from the C4 strain that produces reaction-limiting amounts of an enzyme required for the biosynthesis or addition of mycarose to de-O-methyllactenocin. Thus, the tylosin biosynthetic pathway of Streptomyces fradiae GS52 tends to produce desmycosin rather than the desired tylosin antibiotic compound. Plasmid pHJL284 provides a means for improving the synthetic efficiency of this pathway by providing a non-defective biosynthetic gene and by increasing the copy number of the tylC biosynthetic gene. The concentration of available tylC gene product in the transformed strain, therefore, is increased, resulting in the elevated production of enzyme capable of driving the desired addition reaction. Accordingly, fermentation of Streptomyces fradiae GS52/pHJL284 for 144-168 hours results in a level of tylosin production that is significantly increased over that of the untransformed mutant strain and results in higher tylC enzyme levels than those in the parent C4 strain. Plasmid pHJL284 was also used in the present method to improve the tylosin-producing ability of Streptomyces fradiae GS88, a tylJ mutant, and thus can also be used in the present method to improve the tylosin-producing ability of any organism in which the tylC, tylF, or tylJ gene products (or any combination thereof) are present in rate-limiting amounts for tylosin biosynthesis.

Plasmid pHJL309 contains the tylL and tylM biosynthetic genes and was used in the present method to improve the tylosin-producing ability of Streptomyces fradiae GS33, a tylL mutant, and GS62, a tylM mutant. Plasmid pHJL309 can also be used in the present method to improve the tylosin-producing ability of any organism in which the tylL or tylM gene products (or both) are present in rate-limiting amounts for tylosin biosynthesis.

Plasmid pHJL311 contains the tylC, tylF, tylH, tylJ, and tylK biosynthetic genes and so was used in the present method to improve the tylosin-producing ability of Streptomyces fradiae GS52, a tylC mutant; GS88, a tylJ mutant; GS15 and GS28, both of which are tylF mutants; and GS85, a tylK mutant. Plasmid pHJL311 can also be used in the present method to improve the tylosin-producing ability of any organism in which the tylC, tylF, tylH, tylJ, or tylK gene products (or any combination thereof) are present in rate-limiting amounts for tylosin biosynthesis.

Plasmid pHJL315 contains the tylD, tylE, tylF, tylH, and tylJ biosynthetic genes and so was used in the present method to improve the tylosin-producing ability of Streptomyces fradiae GS48, a tylD mutant;

GS88, a tylJ mutant; GS16, a tylE mutant; GS76, a tylD, tylH double mutant; and GS15 and GS28, both of which are tylF mutants. Plasmid pHJL315 can also be used in the present method to improve the tylosin-producing ability of any organism in which the tylD, tylE, tylF, tylH, or tylJ gene products (or any combination thereof) are present in rate-limiting amounts for tylosin biosynthesis.

These results demonstrate that the vectors of the present invention can increase the antibiotic-producing ability of an antibiotic-producing organism by providing higher enzyme or other gene product levels, as compared to an untransformed organism, of an enzyme or other gene product that is rate-limiting in an antibiotic biosynthetic pathway. However, plasmid maintenance in an antibiotic-producing host cell sometimes requires significant expenditures of the cell's energy, energy that might otherwise be used to produce antibiotic. Thus, certain microorganisms transformed with autonomously replicating vectors actually show a decrease in antibiotic-producing ability, even though the same vectors can increase the antibiotic-producing ability of other organisms. Not wishing the present invention to be bound or limited in any way by theory, this apparent anomaly can be explained by the fact that antibiotics are produced from primary metabolites, such as acetate, propionate, malonyl-CoA, methylmalonyl-CoA, and glucose, by the action of specific enzymes. These enzymes are usually not present during the rapid growth phase of an organism and so do not rob the growing cell of needed compounds. As growth becomes limited by nutritional conditions, antibiotic biosynthetic genes are believed to be activated, causing the synthesis of enzymes that redirect the flow of certain primary metabolites into antibiotic products.

The synthesis of antibiotics is also believed to be a dispensable function in antibiotic-producing organisms because mutants blocked in the biosynthesis of antibiotics are viable and grow as well as the antibiotic-producing parent. Wild-type strains produce a relatively small amount of antibiotic, which is apparently adequate to provide the organism with a selective advantage.

The development of industrial antibiotic producing strains from natural isolates involves many cycles of mutation and selection for higher antibiotic production. Because the synthesis of antibiotics drains primary metabolites and cellular energy away from growth and maintenance functions, it is believed that selection for higher antibiotic production frequently occurs at the expense of the vitality of the organism. Thus, the generation of high antibiotic-producing strains involves finely balancing the cells nutritional and energy resources between growth-maintenance functions and antibiotic production. As a consequence of this fine-tuning, high-yielding production strains tend to be extremely sensitive to factors that affect cellular physiology. For example, introduction of autonomously-replicating vectors, notably multicopy plasmids, sometimes tends to decrease the antibiotic-producing ability of an organism that normally produces antibiotics at high levels. The mechanism of this inhibition is not clear, but may occur at an early step in the biosynthesis of the antibiotic because measurable levels of antibiotic precursors do not accumulate under these conditions. In addition, autonomously replicating vectors may drain pools of precursors for DNA or RNA synthesis or, in high copy number, may titrate DNA binding proteins, such as RNA polymerase, DNA polymerase, polymerase activators, or repressors of gene expression. Another frequent limitation of autonomously replicating vectors is spontaneous loss. Spontaneous loss is especially problematical when the vector reduces growth rate as frequently occurs. Selection for a resistance marker on the plasmid can ensure the growth of homogeneous, plasmid-containing populations but can also disrupt the fine physiological balance (already mentioned) of an antibiotic fermentation. Selection for unstable plasmids operates by killing or inhibiting the bacteria that lose the plasmid and can result in a reduced growth rate.

The negative effect, sometimes observed, of autonomously replicating vectors on the antibiotic-producing ability of a microorganism is greatest in high-producing strains that are delicately balanced with respect to growth-maintenance functions and antibiotic production. The present invention overcomes this previously unrecognized problem of the negative effect of autonomous plasmid replication on high-producing strains by providing methods of culturing the transformed host cell to facilitate identification of transformed cells containing integrated plasmid and, in addition, by providing vectors with features that also facilitate detection of integration. Selecting a culturing procedure that results in integration is important in improving the antibiotic-producing ability of highly selected and conventionally improved antibiotic-producing organisms. Organisms or strains that have a low antibiotic-producing ability can be improved by transformation via either integration or autonomous vector replication. As those skilled in the art of fermentation technology will appreciate, the greatest improvement in antibiotic-producing ability is shown when the present invention is applied to low antibiotic-producing strains.

Integration of plasmid DNA is readily accomplished by transforming a given antibiotic-producing strain or mutant thereof according to standard transformation procedures, selecting or otherwise identifying the transformants, and then culturing the cells under conditions that do not require the presence of plasmid DNA sequences for the host cell to grow and replicate. After several generations under non-selective conditions, certain cells will no longer contain free plasmid DNA, so by selecting for or otherwise identifying

EP 0 238 323 B1

plasmid DNA sequences present in the host cell, one can identify host cells in which the plasmid DNA has integrated into the chromosomal (genomic) DNA of the cell. This culturing technique to obtain integration of vector DNA is especially useful when used in conjunction with a vector that is inherently unstable in the transformed host cell, so that culturing without selective pressure to maintain the vector generates segregants that are free of the plasmid. Bibb et al., 1980, Nature 384:526-531, describe a DNA sequence needed for stable inheritance of a vector, and a variety of vectors have been constructed that lack this stability sequence.

For instance, cloning vectors pHJL210 and pHJL401, which were used to construct the plasmids of the present invention, lack this stability sequence. Plasmid pHJL210 is disclosed in U.S. Patent Application Serial No. 639,566, filed August 10, 1984, (equivalent to European Publication No. 176199). Plasmid pHJL401 is disclosed in U.S. Patent Application Serial No. 841,920, filed March 20, 1986, which is a continuation-in-part of Serial No. 763,172, filed August 7, 1985, (equivalent to European Application No. 86306011.7, filed August 5, 1986, Pub. 213,779). As used, "unstable" refers to plasmids that are lost at high frequency by transformed cells only when those cells are cultured in the absence of selective pressure for plasmid maintenance because, for example, plasmids such as pHJL210 and pHJL401 are quite stable when selective pressure is applied to the transformed host cell. When host cells transformed with stable vectors are cultured in the absence of selective pressure, the vector is not lost with the high frequency observed with unstable vectors, and identification of integrants is made difficult by the great number of cells that still contain autonomously replicating plasmid even after growth under nonselective conditions. Selection for integrants is more fully described below. Once the vector DNA has integrated into the chromosomal DNA of the host cell, one observes the maximum increase in antibiotic-producing ability for that host cell, because inhibition by autonomously replicating plasmid no longer occurs.

Integration of vectors containing cloned genes into the genome of the producing organism can be achieved in a number of ways. One way is to use a lysogenic bacteriophage or other phage vector that can integrate into the genome of the host strain. Another approach is to use a plasmid vector carrying the cloned genes and to screen for integration of the recombinant plasmid into the host genome by a single recombination event between the cloned sequence and the homologous chromosomal sequence. Integration frequency of a vector can be dramatically increased by adding DNA homologous to the genomic DNA of the host cell to the vector. As used "integration" refers both to a single recombination event, known as Campbell-type recombination, and also to a double-crossover event, which results in exchange of genetic information between the vector and the chromosome. With double-crossover recombination, only a portion of the vector integrates into the chromosomal DNA.

For example, a plasmid carrying cloned tylosin biosynthetic genes (tyl) could integrate into the Streptomyces fradiae genome by a single crossover between the tyl genes on the plasmid and the homologous tyl genes in the genome. Another option would be to put a non-tyl S. fradiae DNA sequence on the plasmid in addition to the cloned tyl genes and to screen for integration at the locus corresponding to the non-tyl sequence. The latter approach avoids the possible mutagenic effects of integration into the tyl sequences, but if double-crossover recombination is desired, the vector should comprise the antibiotic biosynthetic genes flanked by separate sequences of homologous DNA.

To avoid the potentially adverse effects, however remote, of a recombinant plasmid (either autonomously replicating or integrated) on tylosin production, one can make use of the ability of Streptomyces fradiae to take up tylosin precursors from the culture medium and convert them to tylosin. In one fermentation of a tylosin-producing strain that had been transformed with plasmid pHJL280 and cultured to obtain integrants, only a subpopulation (~18%) of the cells were thiostrepton resistant, which indicates the presence of plasmid pHJL280 sequences. However, this subpopulation contained multiple copies of the genes for two rate-limiting enzymes, demethylmacrocin-O-methyltransferase (DMOMT) and macrocin-O-methyltransferase (MOMT), and consequently elevated (about 9 fold) levels of the two enzymes, and was able to convert all of the normally accumulated demethylmacrocin and macrocin to tylosin (see Table XIV).

Thus, one can develop specific strains of S. fradiae containing multiple copies of rate-limiting genes and high enzyme levels to act as converters of accumulated precursors to tylosin. These converter strains can be used in several different ways: (1) the converter strain can be co-inoculated into the fermentor with the normal production strain at a low ratio of converter:producer; (2) the converter strain can be introduced into a production fermentation culture late in the cycle to convert intermediates; (3) the converter strain can be kept in a separate "reactor", to which the fermentation production broth from the producer strain would be added; or (4) the converter strain can be immobilized on a column, and fermentation broth from the producer strain passed through. Those skilled in the art will recognize that having separate production and converting populations eliminates the adverse effects that recombinant plasmids sometimes have on antibiotic production in high antibiotic-producing strains.

22

Separate populations also eliminate vector stability problems, because the converting strains can be grown in small vessels in which antibiotic selection or some other selection means for maintenance of the plasmid can be carefully regulated and controlled. In essence, the converting strain is a source of enzymes, and the production of these enzymes at high level can be approached in much the same way as production of proteins from recombinant plasmids in E. coli.

Of course, antibiotic production is only increased by the method of the present invention when the transforming DNA comprises a gene that encodes the rate-limiting enzyme of the untransformed strain. Various methods for determining the rate-limiting step in the biosynthesis of an antibiotic are known in the art (Seno and Baltz, 1982, Antimicrobial Agents and Chemotherapy 21:758-763), but there is no need to identify the rate-limiting step when the entire set of antibiotic biosynthetic genes are available for introduction into the antibiotic-producing strain. If a rate-limiting enzyme is not known, the antibiotic-producing strain is transformed with the entire set of antibiotic biosynthetic genes, thus ensuring that, no matter what enzyme is rate-limiting, the transformed host cell will have higher levels of the rate-limiting enzyme than the untransformed host cell. Often, however, the rate-limiting enzyme of an antibiotic biosynthesis pathway will be known, and the method of the present invention can be used to increase the antibiotic-producing ability of the organism by transforming the organism with a vector that encodes the rate-limiting antibiotic biosynthetic enzyme.

For instance, the GS15 strain, which produces no readily detectable tylosin (the level of tylosin produced by these cells is below the detection limits for the assay used to determine tylosin levels) and the GS28 strain, which produces very low levels of tylosin, contain tylF mutations, so that it is a relatively simple matter to identify the rate-limiting step in tylosin biosynthesis in these mutant strains. The strain from which the GS15 and GS28 strains were derived, designated Streptomyces fradiae C4, produces high levels of tylosin and accumulates relatively large amounts of macrocin, the immediate precursor of tylosin on which the tylF gene product acts to form tylosin. Other S. fradiae strains that produce even more tylosin than the C4 strain accumulate even more macrocin than the C4 strain. These observations indicate that the tylF gene product is present in rate-limiting amounts for the biosynthesis of tylosin in high tylosin-producing strains. Transformation of these macrocin-accumulating strains with a vector comprising the tylF gene followed by isolation of those transformants that only contain integrated copies of the vector yields transformants that produce more tylosin than the untransformed cells. The increase in tylosin production observed in these transformants is related to the amount of macrocin that accumulates in the untransformed cells. It will be apparent to those skilled in the art that the transformants produced by the foregoing procedure might still contain rate-limiting amounts of the tylF gene product, in which case a further increase of the tylF copy number would further increase tylosin yield, or the transformed strains might now contain rate-limiting amounts of yet another antibiotic biosynthetic enzyme, the level of which could be made non-rate-limiting by the method of the present invention.

The present invention provides both a method and recombinant DNA cloning vectors for increasing the production of an antibiotic by manipulation of antibiotic biosynthetic pathways. An illustrative antibiotic biosynthetic pathway involves the biosynthesis of tylosin, a complex macrolide produced by strains of Streptomyces fradiae, Streptomyces rimosus, and Streptomyces hygroscopicus. Tylosin is composed of a 16-member branched lactone (tylonolide) to which three sugars (mycarose, mycaminose, and mycinose) are attached. The lactone is derived from two acetates, five propionates, and a butyrate by condensation of a propionyl-S-coenzyme A molecule with two malonyl-S-coenzyme A molecules, four methylmalonyl-S-coenzyme A molecules, and an ethylmalonyl-S-coenzyme A molecule by a scheme believed analogous to that involved in fatty acid biosynthesis. Lactone formation, sugar biosynthesis/attachment, and the conversion of resultant intermediate compounds to tylosin are catalyzed by a series of gene-encoded enzymes. Cloning genes that code for such enzymes allows modification and improvement in the operational efficiency of the tylosin biosynthetic pathway and is illustrative of the present invention.

Illustrative tylosin biosynthetic genes that can be used for purposes of the present invention include, for example, the tylC, tylD, tylE, tylF, tylH, tylJ, tylK, tylL, and tylM, genes. Of this group, the tylF gene is preferred because the macrocin O-methyltransferase enzyme encoded thereby appears to be rate-limiting if the tylosin biosynthetic pathway of most tylosin-producing strains. Macrocin accumulates to unacceptable levels under conditions of optimum fermentation of Streptomyces fradiae because of the rate-limiting steps catalyzed by the tylF gene product. The tylF enzyme catalyzes the conversion of macrocin to tylosin, as depicted in Figure 1 of the accompanying drawings. Over-production of the tylF gene product, macrocin O-methyltransferase, results in the more efficient operation of the tylosin biosynthetic pathway as indicated by increased antibiotic yield and lower cost of fermentation.

Those skilled in the art will recognize that the present invention is not limited to the use of plasmids pHJL280, pHJL284, pHJL309, pHJL311, or pHJL315. The antibiotic biosynthetic genes contained in these vectors can be excised in whole or in part and ligated into any number of different recombinant DNA cloning vectors. For instance, digestion of plasmid pHJL280 with restriction enzymes BamHI and BglII yields five BamHI-BamHI fragments with sizes of ~10.3 kb, ~6.54 kb, ~2.3 kb, ~1.7 kb, and ~1.0 kb; two BamHI-BglII fragments with sizes of ~2.9 kb and 2.0 kb; and one BglII-BglII fragment ~0.2 kb in size. The ~2.9 kb BamHI-BglII fragment of plasmid pHJL280 contains the tylF gene. Digestion of plasmid pHJL280 with restriction enzymes BglII and EcoRI generates four fragments: an ~11.24 kb EcoRI-EcoRI fragment; an ~11.5 kb BglII-EcoRI fragment; an ~4.0 kb EcoRI-BglII fragment, and an ~0.2 kb BglII-BglII fragment. The ~4.0 kb EcoRI-BglII fragment of plasmid pHJL280 contains the tylE gene.

Digestion of plasmid pHJL284 with restriction enzymes BamHI and EcoRI generates three BamHI-BamHI fragments with sizes of ~9.7 kb, ~2.3 kb, and ~1.0 kb; and four EcoRI-BamHI fragments with sizes of ~6.24 kb, ~4.3 kb, ~2.3 kb, and ~1.1 kb. The ~2.3 kb BamHI-EcoRI fragment of plasmid pHJL284 contains the tylF gene. Digestion of plasmid pHJL284 with restriction enzyme EcoRI generates two fragments with sizes of ~16.4 kb and ~10.54 kb; the ~16.4 kb fragment contains the tylF, tylC, and tylJ genes. The ~1.7 kb EcoRI-BamHI restriction fragment of plasmid pHJL311 comprises the tylK gene. The ~18.5 kb EcoRI restriction fragment, as well as the ~8.3 kb BamHI-KpnI restriction fragment, of plasmid pHJL309 contains the tylL and tylM genes.

Any of the aforementioned tyl gene-containing fragments can be ligated into other vectors to make vectors useful in the present method. Such other vectors include, for example, those vectors disclosed in U.S. Patent Nos. 4,468,462; 4,513,086; 4,416,994; 4,503,155; and 4,513,185; and also plasmids pIJ101, pIJ350, pIJ702 (ATCC 39155), SCP2* (NRRL 15041), pHJL192, pHJL197, pHJL198, pHJL210, pHJL211, pHJL400, pHJL401, pHJL302, pIJ922, pIJ903, pIJ941, pIJ940, and pIJ916. These vectors replicate in Streptomyces fradiae and other tylosin-producing strains and are thus useful for cloning the present antibiotic biosynthetic genes. The "unstable" vectors described above are preferred when integration of the vector is desired.

Illustrative Streptomyces strains that can be used for purposes of the present invention include, for example, S. fradiae, S. fradiae GS52, S. fradiae GS48, S. fradiae GS16, S. fradiae GS28, S. fradiae GS15, S. fradiae GS76, S. rimosus, and S. hygroscopicus. Streptomyces hygroscopicus and S. rimosus are well known, having been deposited at the American Type Culture Collection (ATCC), Rockville, Maryland 20852. A number of strains of S. hygroscopicus can be obtained under the accession numbers ATCC 27438, ATCC 21449, ATCC 15484, ATCC 19040, and ATCC 15420, and S. rimosus can be obtained under the accession number ATCC 10970. Of the Streptomyces taxa, S. fradiae GS16, S. fradiae GS15, and S. fradiae GS28 are preferred, especially for transformation with plasmid pHJL280. Streptomyces fradiae is also an especially well known microorganism and several strains are available, on an unrestricted basis, from the Northern Regional Research Laboratory (NRRL), Peoria, Illinois 61604 and the ATCC under the respective accession numbers NRRL 2702, NRRL 2703, and ATCC 19609.

The recombinant plasmids described in the present invention each comprise one or more antibiotic biosynthetic genes. Unless part of a polycistron, each antibiotic biosynthetic gene normally comprises: (1) a promoter that directs transcription of the gene; (2) a sequence that, when transcribed into mRNA, directs translation of the transcript; (3) a protein-coding sequence; and (4) a transcription terminator. Each of these elements is independently useful and can, through the techniques of recombinant DNA technology, be used to form recombinant genes of great variety. As one example, the protein-coding sequence for the tylF gene can be linked to the promoter, translation-activating sequence, and transcription-terminating sequence from a non-Streptomyces fradiae gene to form a recombinant gene that functions in the host from which the non-S. fradiae sequences were isolated. Such a novel gene could be used to produce a hybrid antibiotic if introduced into an organism that produced an antibiotic or antibiotic intermediate that is not found in the tylosin pathway but that would serve as a substrate for the novel gene product. Similarly, the promoter and other regulatory elements of the tylF gene could be linked to the coding sequence of a non-tylosin antibiotic biosynthetic gene to prepare a hybrid gene that would function in S. fradiae. Thus, the individual elements of each of the antibiotic biosynthetic genes on each of the plasmids described herein comprise an important component of the present invention.

For example, sequence data on the tylF nucleotide sequence has identified the tylF promoter. The sequence (only one strand of which is depicted for convenience) is shown below; the promoter and translation-activating sequence of the tylF gene are believed to reside in the sequence between residues 1 and 207. The sequence ends with the beginning of the coding region of the tylF gene.

As an additional embodiment, the gene sequence for the tylF gene is provided. In particular, the entire tylF gene, including the promoter and translation-activating sequence noted above, has been found to have the following sequence:

24

EP 0 238 323 B1

```
             10              20              30              40
5'-TTC GCG GGA TGG ATG CTG ACC CGG GGG TCG GCC AGC AGC GCC CGG ACG


     50              60              70              80              90
   TGA TCT GGC GGG AGA TCA GCC AGA CCG GCG CCC CGT CCC ACA GCT CGG


       100             110             120             130             140
   CCC GGG CGA TCG GCT CCT CCG CCC GGA GGG CGG CGT ACT GCT CGG GAG


       150             160             170             180             190
   GGC TGA AGG GAC AGG TGC GGG CGA CCG GCC AGG CGA TGC TGC GCC GGC


         200             210             220             230             240
   CTG CGG CCC CGT CGG TGT CGT TGG CGC GTG CTG CGG GCA ACA GAA TCC


         250             260             270             280
   CCT TTT GTG ACG GGC GGG CGT CCC CGG ACG AGG ACA CGA CTC GCT GCG


     290             300             310             320             330
   GCC TCA ACG AAA ACA CCG TGT CCG GTG CCC AGG CCA CGA ACG GTG ACC


       340             350             360             370             380
   GGT CTG TGT CAG GTC GCC CGT GGT GAC GGG CTC CGG GGC GGC GGC GCG
```

25

```
        390             400             410             420             430
GGC GGC CGA CCT TGA CAT ACC CGC GGC CGG GCT CCT CGT TCC GGC GCG


        440             450             460             470             480
GCC CGC GCC GAT AGC GTC CGT CCT CAC CGG CTC CGG CGT CCG CGT CCC


            490             500             510             520
CGC CGG GAC GTG CCA CCT CTC CCG ACC CCG CGA GCC GAT CGA CCC GCT


    530             540             550             560             570
ACT GGA GGA CCC GTG GCA CCT TCC CCG GAC CAC GCC CGC GAT CTC TAC
                VAL ALA PRO SER PRO ASP HIS ALA ARG ASP LEU TYR
                                     5                   10


        580             590             600             610             620
ATC GAG CTG CTG AAG AAG GTC GTC TCG AAC GTC ATC TAC GAG GAC CCC
ILE GLU LEU LEU LYS LYS VAL VAL SER ASN VAL ILE TYR GLU ASP PRO
                15                  20                  25


        630             640             650             660             670
ACC CAT GTG GCG GGG ATG ATC ACC GAC GCG TCG TTC GAC CGG ACG TCC
THR HIS VAL ALA GLY MET ILE THR ASP ALA SER PHE ASP ARG THR SER
        30                  35                  40


        680             690             700             710             720
CGT GAG AGC GGC GAG GAC TAC CCC ACG GTC GCC CAC ACG ATG ATC GGC
ARG GLU SER GLY GLU ASP TYR PRO THR VAL ALA HIS THR MET ILE GLY
45                  50                  55                  60
```

```
          730            740            750            760
CTC AAG CGT CTG GAC AAT CTC CAC CGG TGC CTC GCG GAC GTC GTG GAG
LEU LYS ARG LEU ASP ASN LEU HIS ARG CYS LEU ALA ASP VAL VAL GLU
            65                       70                       75


 770            780            790            800            810
GAC GGC GTC CCC GGT GAC TTC ATC GAG ACC GGG GTG TGC CGC GCG CCG
ASP GLY VAL PRO GLY ASP PHE ILE GLU THR GLY VAL CYS ARG ALA PRO
            80                       85                       90


     820            830            840            850            860
TGC ATC TTC GCC CGC GGA CTG CTG AAC GCG TAC GGC CAG GCC GAC CGC
CYS ILE PHE ALA ARG GLY LEU LEU ASN ALA TYR GLY GLN ALA ASP ARG
            95                      100                      105


     870            880            890            900            910
ACC GTC TGG GTC GCC GAC TCC TTC CAG GGC TTT CCC GAG CTG ACC GGG
THR VAL TRP VAL ALA ASP SER PHE GLN GLY PHE PRO GLU LEU THR GLY
            110                     115                      120


         920            930            940            950            960
TCC GAC CAC CCG CTG GAC GTC GAG ATC GAC CTC CAC CAG TAC AAC GAG
SER ASP HIS PRO LEU ASP VAL GLU ILE ASP LEU HIS GLN TYR ASN GLU
125                      130                      135                      140


         970            980            990           1000
GCC GTG GAC CTG CCC ACC AGC GAG GAG ACC GTG CGG GAG AAC TTC GCC
ALA VAL ASP LEU PRO THR SER GLU GLU THR VAL ARG GLU ASN PHE ALA
            145                     150                      155
```

27

```
    1010        1020          1030         1040          1050
CGG TAC GGG CTG CTC GAC GAC AAC GTC CGT TTC CTG GCG GGG TGG TTC
ARG TYR GLY LEU LEU ASP ASP ASN VAL ARG PHE LEU ALA GLY TRP PHE
            160                   165               170


    1060        1070          1080         1090          1100
AAG GAC ACC ATG CCG GCT GCG CCC GTG AAG CAG CTC GCG GTG ATG CGC
LYS ASP THR MET PRO ALA ALA PRO VAL LYS GLN LEU ALA VAL MET ARG
            175                   180               185


    1110        1120          1130         1140          1150
CTG GAC GGC GAC TCC TAC GGC GCC ACC ATG GAT GTG CTC GAC AGC CTG
LEU ASP GLY ASP SER TYR GLY ALA THR MET ASP VAL LEU ASP SER LEU
        190                195               200


        1160        1170          1180         1190          1200
TAC GAG CGG CTG TCG CCG GGC GGT TAC GTC ATC GTC GAC GAC TAC TGC
TYR GLU ARG LEU SER PRO GLY GLY TYR VAL ILE VAL ASP ASP TYR CYS
205                 210               215               220


        1210        1220          1230         1240
ATC CCG GCC TGC CGC GAG CGG TGC ACG ACT TCC GCG ACC GGC TCG GCA
ILE PRO ALA CYS ARG GLU ARG CYS THR THR SER ALA THR GLY SER ALA
                225               230               235


    1250        1260          1270         1280          1290
TCC GCG ACA CGA TCC ACC GGA TCG ACC GCC AGG GCG CTA TTG GCG GCA
SER ALA THR ARG SER THR GLY SER THR ALA ARG ALA LEU LEU ALA ALA
            240                   245               250
```

28

EP 0 238 323 B1

```
      1300          1310          1320          1330          1340
CAG CGG CTG AGT CGT TCC GCC CGA GAG CCC GAC GAG AGC AGG AGA TAT
GLN ARG LEU SER ARG SER ALA ARG GLU PRO ASP GLU SER ARG ARG TYR
      255                       260                       265


      1350          1360          1370          1380          1390
GCG AGA CAC GAC GCG CCC GCT CGG CAT TGA GGG AGC GTG GGT GAT CCA
ALA ARG HIS ASP ALA PRO ALA ARG HIS
      270                       275


      1400          1410          1420          1430          1440
GCC GGA GAT CCA TCC GGA CCG GCG CGG CGA GTT CCA CGC GTG GTT CCA


      1450          1460          1470          1480
GAG CCA GCC GAG TTC CGG CGG CTG ACC GGT CAC TCC TTC TCC GTG CCG


1490          1500          1510          1520          1530
CAG GTC GTC AAT ATC GCG TGT CCC GGA AAG GCG CCG CTG CGG CAT CCA


      1540          1550          1560          1570          1580
CTT CTG CCG AGG TGC CAC CGG GCC GAG GCC AAG TAC AGC GGC GTG TGT


      1590          1600          1610          1620          1630
GCA GGG CGC CGG TGT CGA GGT CGT CGT CGA CGC GCC GGT GTC GAG GTC


      1640
GTC GTC GAC-3'
```

wherein A is deoxyadenyl residue; G is a deoxyguanyl residue; C is a deoxycytidyl residue; and T is a deoxythymidyl residue. The structural gene, as indicated above, begins at residue 541 and continues through to residue 1371, terminating with the stop codon located at residue 1372. The amino acid sequence of the tylF structural gene is that indicated under the corresponding nucleotide sequence. As those skilled in the art will recognize, because of the degeneracy of the genetic code, equivalent sequences to that specifically provided above can be obtained which will encode the same tylF gene product. The scans for obtaining such equivalent sequences will be familiar to those skilled in the art. Further, that a specific sequence is provided is not to be construed as limiting the invention in any way.

Streptomyces fradiae strains can be cultured in a number of ways using any of several different media. Carbohydrate sources that are preferred in a culture media include, for example, molasses, glucose, dextran, and glycerol, and nitrogen sources include, for example, soy flour, amino acid mixtures, and peptones. Nutrient inorganic salts are also incorporated into the medium and include the customary salts capable of yielding sodium, potassium, ammonium, calcium, phosphate, chloride, sulfate, and like ions. As is necessary for the growth and development of other microorganisms, essential trace elements are also

29

added. Such trace elements are commonly supplied as impurities incidental to the addition of other constituents of the medium. S. fradiae strains are grown under aerobic culture conditions over a relative wide pH range of about 5.5 to 8 at temperatures ranging from about 25° to 37°C. In particular, tylosin can be produced by cultivation of tylosin-producing strains of, for example, S. fradiae such as those containing the vectors provided by the present invention. The culture medium employed can be any one of a number of media since the organism is capable of utilizing many energy sources. However, for economy of production, maximum yields of antibiotic, and ease of isolation of the antibiotic, certain culture media are preferable. The media which are useful in the production of tylosin include an assimilable source of carbon such as glucose, sucrose, fructose, starch, glycerine, molasses, dextrin, brown sugar, corn steep solids, and the like. The preferred sources of carbon are glucose and starch. Additionally, employable media include a source of assimilable nitrogen such as linseed meal, tankage, fish meal, cotton seed meal, oatmeal, ground wheat, soybean meal, beef extract, peptones (meat or soy), casein, amino acid mixtures, and the like. Preferred sources of nitrogen are soybean meal, casein, and corn steep solids.

Mineral salts, for example, those providing sodium, potassium, ammonium, calcium, magnesium, cobalt, sulfate, chloride, phosphate, carbonate, acetate, and nitrate ions, and a source of growth factors such as distillers' solubles and yeast extract, can be incorporated into the media with beneficial results.

As is necessary for the growth and development of other microorganisms, essential trace elements should also be included in the culture medium for growing the microorganisms employed in this invention. Such trace elements are commonly supplied as impurities incidental to the additional of the other constituents of the medium.

The initial pH of the culture medium can be varied widely. However, it has been found that the initial pH of the medium desirably is between about pH 5.5 and about pH 8.0, and preferably is between about pH 6.5 and about pH 7.0. As has been observed with other organisms, the pH of the medium gradually increases throughout the growth period of the organism during which time tylosin is produced, and may attain a pH from about pH 7.2 to about pH 8.0 or above, the final pH being dependent at least in part on the initial pH of the medium, the buffers present in the medium, and the period of time the organism is permitted to grow.

Submerged, aerobic cultural conditions are the conditions of choice for the production of large amounts of tylosin. For preparation of relatively small amounts, shake flasks and surface culture in bottles can be employed, but for the preparation of large amounts, submerged aerobic culture in sterile tanks is preferred. The medium in the sterile tank can be inoculated with a sporulated suspension. However, because of the growth lag experienced when a sporulated suspension is used in the inoculum, the vegetative form of the culture is preferred to avoid the pronounced growth lag, thereby permitting a more efficient use of the fermentation equipment Accordingly, it is desirable first to produce a vegetative inoculum of the organisms by inoculating a relatively small quantity of culture medium with the spore form of the organism, and when a young, active, vegetative inoculum has been obtained, to transfer the vegetative inoculum aseptically to the large tank. The medium in which the vegetative inoculum is produced can be the same or a different medium than that utilized for the large scale production of tylosin.

The organisms grow best at temperatures in a range of about 25°C to about 37°C. Optimal tylosin production appears to occur at a temperature of about 26-30°C.

As is customary in submerged culture processes, sterile air is blown through the culture medium. For efficient growth of the organism and tylosin production, the volume of air employed in the tank production of tylosin preferably is upwards of 0.1 volume of air per minute per volume of culture medium. Efficient growth and optimal yields of tylosin are obtained when the volume of air used is at least one volume of air per minute per volume of culture medium.

The concentration of tylosin activity in the culture medium can readily be followed during the fermentation period by testing samples of the culture medium for their inhibitory activity against the growth of an organism known to be inhibited in the presence of tylosin.

In general, after inoculation, maximum production of tylosin occurs within about 2 to 7 days when submerged aerobic culture or shake flask culture is employed, and within about 5 to 10 days when surface culture is used.

If desired, the mycelium and undissolved solids are removed from the fermentation broth by conventional means such as filtration or centrifugation. If desired, the tylosin is removed from the filtered or centrifuged broth by employing adsorption or extraction techniques familiar to those skilled in the art.

For the extraction of tylosin from the filtered broth, water-immiscible, polar, organic solvents are preferred, such including esters of fatty acids, for example, ethyl acetate and amyl acetate; chlorinated hydrocarbons, for example, chloroform ethylene dichloride, and trichloroethylene; water-immiscible alcohols, for example, butyl and amyl alcohols; water-immiscible ketones, for example, methyl isobutyl ketone and methyl amyl ketone; and others, for example, diethyl ether and methyl propyl ether. Other solvents of similar character can also be employed. Chloroform and amyl acetate are the presently preferred extraction solvents.

For the recovery of tylosin by adsorption techniques, various absorbants and ion exchange resins can be used, for example, carbon, silica gel, alumina, and ion exchange resins of acidic character such as "XE" 64 and "IRC" 50 (weakly acidic cation exchange resins sold by Rohm & Haas Company), carboxymethyl cellulose resin, and "Dowex" 50 (a strongly acidic cation exchange resin sold by The Dow Chemical Company). The tylosin can be adsorbed on one of the above or similar adsorbents from a solution in chloroform, acetone, benzene or other suitable solvents. The adsorbed tylosin can then be suitable solvents. The adsorbed tylosin can then be eluted from the adsorbent by suitable elution techniques such as by washing the adsorbent on which the tylosin is adsorbed, with a lower alcohol, for example, methanol or ethanol, or with a lower alcohol containing up to about 50 percent of a lower ketone, for example, acetone.

The organic solvent extract obtained by the preferred extraction method can be directly evaporated to dryness to provide crude tylosin. Alternatively the organic solvent extract can be used to provide purified tylosin by concentration in vacuo the organic solvent extract of tylosin by decolorizing the concentrate with carbon, and by precipitating the tylosin by the addition of a non-polar solvent, for example, petroleum ether. The precipitate which is thus obtained is a solid, purified tylosin which is usually amorphous. The amorphous precipitate can be crystallized by employing one of the crystallizing solvents mentioned above. Alternatively, tylosin can be recovered from a tylosin-containing organic extract, by adsorption chromatography, and by recovery of the absorbed tylosin from the absorbent by elution.

Other means for preparing the desired product from the culture medium will be recognized by those skilled in the art.

The acid addition salts of tylosin can be formed with mineral acids, for example, sulfuric, hydrochloride and nitric acid, and with organic acids, for example, tartaric, gluconic, oxalic and acetic acid. The acid addition salts can be prepared by dissolving the free base of tylosin in a solvent in which it is soluble, such as acetone or ether, and adding to the solution an equimolar amount of the appropriate acid. The salt which is formed usually precipitates out of solution. In the event the salt does not precipitate, it can be recovered by evaporating the solution to a smaller volume to permit precipitation, or by adding a miscible solvent in which the salt is not soluble.

The following non-limited are provided to further illustrate the invention. Sources of reagents are provided merely for convenience and in no way limit the invention.

Example 1

Isolation of Plasmid pHJL280

## A.   Culture of E. coli K12 HB101/pHJL280

Lyophils of E. coli K12 HB101/pHJL280 can be obtained from the NRRL under the accession number NRRL B-18043. The lyophilized cells are streaked onto L-agar plates (L agar contains 10 g of Bacto Tryptone, 5 g of Bacto Yeast Extract, 10 g of NaCl, 2 g of glucose, and 15 g of agar per liter) containing 50 μg/ml ampicillin to obtain a single-colony isolate of E. coli K12 HB101/pHJL280. One such colony was used to inoculate 100 ml of L broth (L broth is L agar without the agar), which was then incubated aerobically at 37°C overnight (about 16 hours). The following morning, the cells were harvested by centrifugation at 10,000Xg for 10 minutes. The ~1 g of cells obtained by this procedure are used to prepare plasmid pHJL280 DNA in substantial accordance with the procedure described below.

B. Plasmid Isolation

The cell pellet obtained in Example 1A was resuspended in 10 ml of a solution composed of 25% sucrose and 50 mM Tris-HCl at a pH = 8.0. About 1 ml of a 10 mg/ml solution of lysozyme in 50 mM Tris-HCl at a pH = 8.0 was added to the cell suspension, and the resulting mixture was incubated on ice for 5 minutes. About 4 ml of 0.25 M EDTA, pH = 8.0, were then added to the cell suspension, and incubation on ice was continued for another 5 minutes. About 16 ml of lysis solution (lysis solution contains 0.4% deoxycholate; 1% Brij58 (Sigma Chemical Co., P.O. Box 14508, St. Louis, MO 63178); 0.05 M Tris-HCl, pH = 8.0; and 0.0625 M EDTA) were added to the lysozyme-treated cells, and the resulting mixture was

31

incubated at 37°C for 15 minutes.

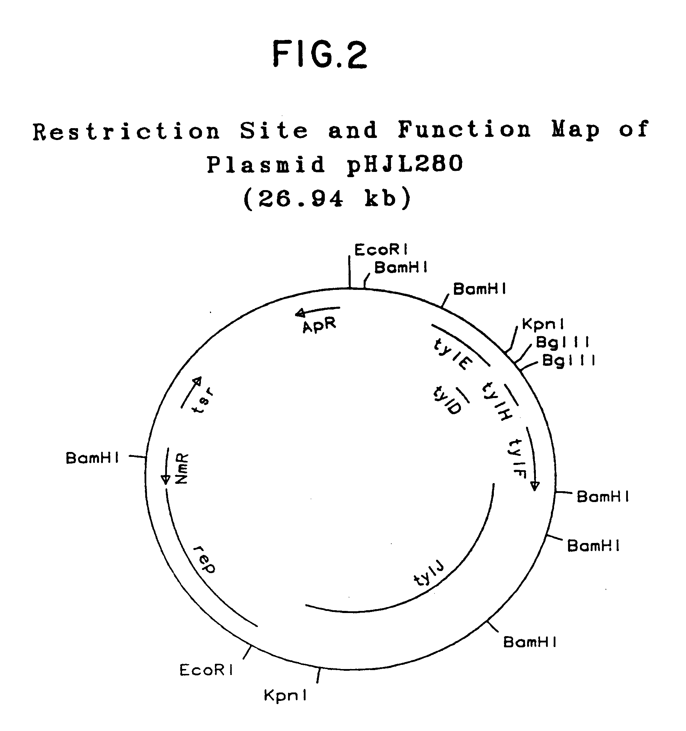

The cell lysate was cleared by centrifugation at 48,000Xg for 25 minutes. The supernatant was decanted into a separate tube, to which was added 0.1 volume, of 3.0 M NaOAc at a pH = 8.0 and 0.64 volume of isopropyl alcohol. The DNA precipitate was collected by centrifugation at 20,000Xg for 10 minutes and then redissolved in 0.1 volume of TE buffer (10 mM Tris-HCl, pH = 7.8, and 1 mM EDTA). The solution of DNA was incubated at 65°C for 30 minutes and then purified by equilibrium-density-gradient ultracentrifugation in CsCl and propidium diiodide. The plasmid pHJL280 DNA obtained by this procedure was dissolved in TE buffer at a concentration of about 1 μg/μl. A restriction site map of plasmid pHJL280 is presented in Figure 2 of the accompanying drawings.

Example 2

Isolation of Plasmids pHJL284, pHJL309, pHJL311, and pHJL315

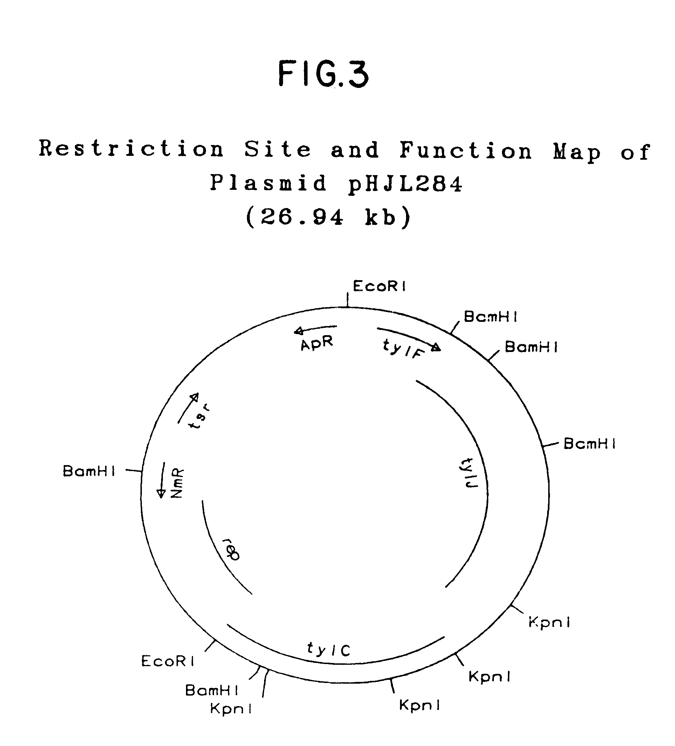

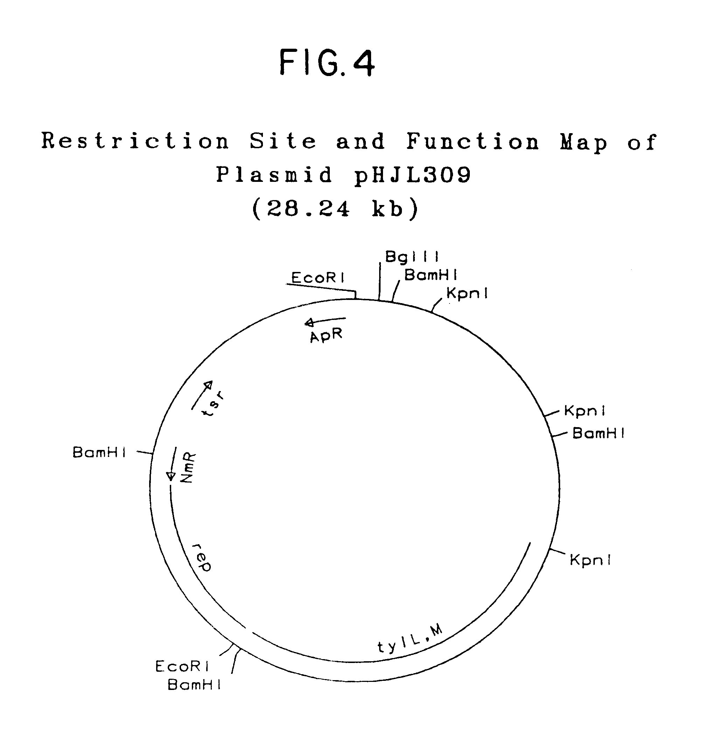

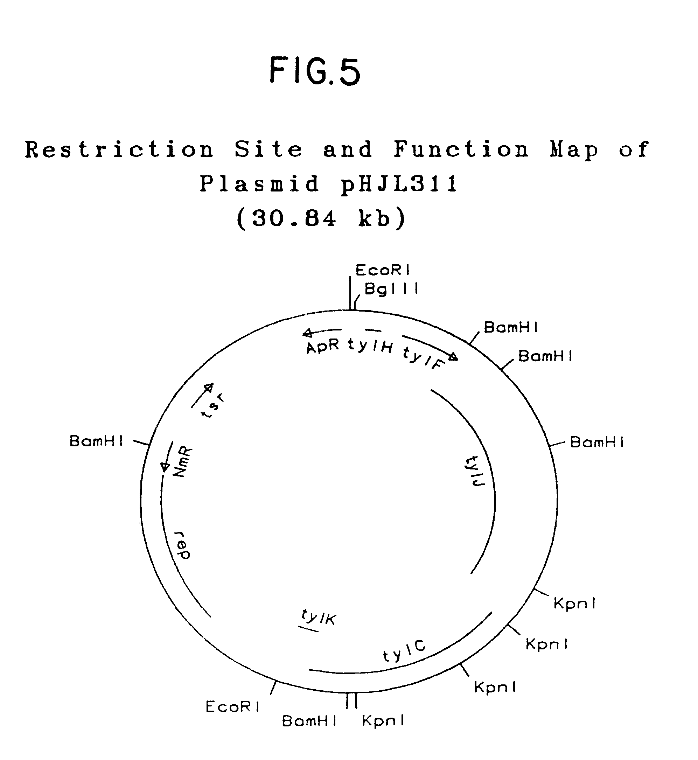

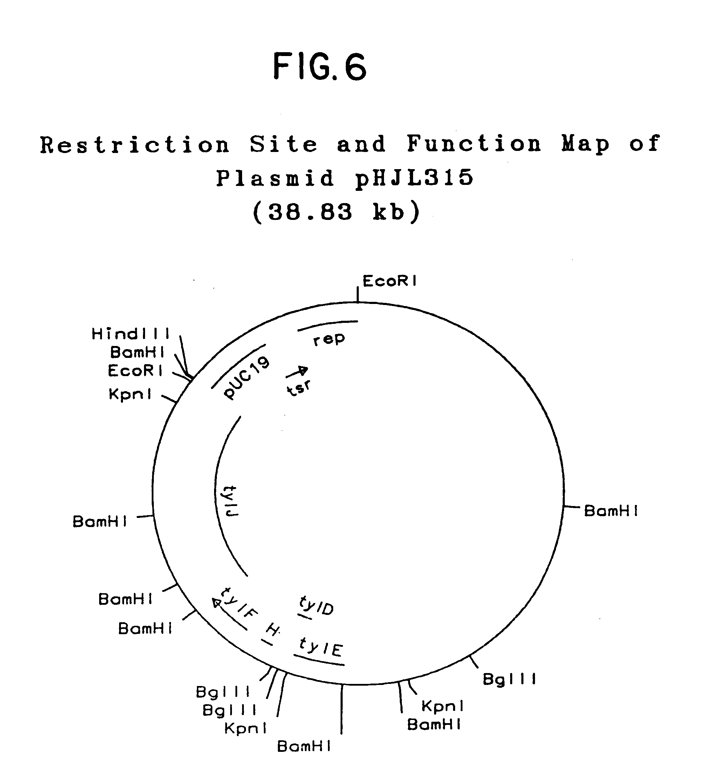

Lyophils of the E. coli strains harboring plasmids pHJL284, pHJL309, pHJL311, and pHJL315 can be obtained from the NRRL under the accession numbers listed in Table X. The desired plasmids are each obtained and purified from the lyophilized cells in substantial accordance with the teaching of Example 1. Restriction site maps of the plasmids are presented in Figures 2-6 of the accompanying drawings.

Example 3

## Construction of _Streptomyces fradiae_ GS28/pHJL280

A culture of Streptomyces fradiae GS28 was inoculated into 20 ml of trypticase-soya broth (TSB) and incubated in a water-bath incubator at 29°C at 260 rpm overnight (about 16 hours). The culture was homogenized using a homogenizing vessel (Thomas Scientific, Swedesboro, NJ) and a T-Line laboratory stirrer and then fragmented using a Sonifier Cell Disruptor (Heat Systems Ultrasonics, Inc.) for 7 seconds at 76 Watts. Four ml of the homogenized, fragmented culture were inoculated into 20 ml of TSB (BBL) containing 0.3% weight by volume glycine, and the culture was again incubated overnight at 29°C. The following morning, the culture was homogenized and recultured as described above. After this third overnight incubation, the culture was homogenized, collected, and then washed twice with P media. P media was prepared by adding 103 g of sucrose to 0.25 g of $K_2SO_4$ and 2.03 g of $MgCl_2$-$6H_2O$ and then adding deionized water to a final volume of 700 ml. The mixture was then sterilized, and to each 70 ml of solution, about 10 ml each of 0.05 g $KH_2PO_4$/100 ml of deionized water; 2.78 g $CaCl_2$/100 ml of deionized water; and 0.25 M TES (2-([tris-(hydroxymethyl)methyl]-amino)ethanesulfonic acid)) at a pH = 7.2 were added.

The cell pellet was resuspended in 15 ml of P media containing 1 mg/ml lysozyme (Calbiochem, La Jolla, CA 92037) and then incubated at room temperature for about one-and-one-half hours to form protoplasts. The protoplasts were gently collected by centrifugation, washed twice with P media, resuspended in 2 ml of P media, and incubated on ice until use. About 1 μg of plasmid pHJL280 DNA was added to about 50 μl of 1 mg/ml heparin sulfate (Sigma) and incubated on ice for about 10 minutes. Much less plasmid DNA, about 5-100 nanograms, can be used to transform Streptomyces fradiae if prepared from a S. fradiae host. The procedure for isolating Streptomyces plasmid DNA is described in Hopwood et al., 1985, Genetic Manipulation of Streptomyces: A Laboratory Manual (John Innes Foundation, Norwich, England). The DNA/heparin solution was first added to about 200 μl of protoplasts, and about 0.9 ml of a solution composed of 55% PEG 1000 (Sigma) in P medium was then added to the DNA/protoplast mixture, and the resulting mixture was gently mixed at room temperature.

The mixture was plated in varying aliquots onto R2 plates using 4 ml of soft-R2-agar overlays. R2 plates contain 30 ml of R2 media and have been dried at 37°C for about 4 days. R2 media is prepared by adding 103 g sucrose, 0.25 g $K_2SO_4$, 2 ml of trace element solution, 10.12 g $MgCl_2$-$6H_2O$, 10.0 g of glucose, 2.0 g of L-asparagine, 0.1 g of Casamino acids, and 22 g of agar to 700 ml of water; sterilizing the resulting solution; and finally, adding 100 ml of each of the following solutions: 0.05 g $KH_2PO_4$/100 ml of deionized water; 2.22 g $CaCl_2$/100 ml of deionized water; and 0.25 M TES, pH = 7.2. The pH of the final solution is adjusted to equal 7.2. Trace element solution contains 40 mg $ZnCl_2$, 200 mg $FeCl_3$-$6H_2O$, 10 mg $CuCl_2$-$2H_2O$, 10 mg $MnCl_2$-$4H_2O$, 10 mg $Na_2B_4O_7$-$10H_2O$, and 10 mg $(NH_4)_6Mo_7O_{24}$•$4H_2O$ per liter. The soft-R2-

32

agar overlays are prepared by adding 51.5 g of sucrose, 5.06 g $MgCl_2$-$6H_2O$, 1.11 g $CaCl_2$, 50 ml of 0.25 M TES at a pH = 7.2, and 2.05 g agar to enough deionized water to achieve a final volume of 500 ml. The mixture is steamed to melt the agar, decanted into 4 ml aliquots, and autoclaved prior to use. After the transformed protoplasts had been plated, the plates were incubated at 29°C for 24 hours, and then, 4 ml of soft-R2 agar containing 25 $\mu$l of 50 mg/ml thiostrepton (E. R. Squibb, Princeton, NJ 08540) were spread over the protoplasts. Incubation of the plates at 29°C was continued until regeneration was complete, usually a period of about 7-14 days, to select for the desired S. fradiae GS28/pHJL280 transformants.

The Streptomyces fradiae GS28/pHJL280 strain was cultured and produced macrocin O-methyltransferase and tylosin at levels above that produced in the untransformed S. fradiae GS28 strain. Macrocin O-methyltransferase activity was assayed and determined in substantial accordance with the teaching of Yeh et al., 1984, Journal of Chromatography 288:157-165. Comparison of the macrocin O-methyltransferase activities in the transformed, GS28/pHJL280, and parental, GS28, strains showed a 60-to-100-fold increase of enzyme and 14-to-18-fold increase of tylosin production in the transformed strain. Tylosin production was assayed and determined in substantial accordance with the teaching of Baltz and Seno, 1981, Antimicrobial Agents and Chemotherapy 20:214-225; and Kennedy, J.H., 1983, Journal of Chromatography 281:288-292.

Example 4

## Construction of Streptomyces fradiae GS15/pHJL280

The desired strain was constructed in substantial accordance with the teaching of Example 3 except that Streptomyces fradiae GS15, rather than S. fradiae GS28, was used. The desired strain was cultured for 72 hours and produced macrocin O-methyltransferase and tylosin at levels above that produced in the untransformed S. fradiae GS15 strain, which produces no readily detectable tylosin.

Example 5

## Construction of Streptomyces fradiae GS15/pHJL284

The desired strain was constructed in substantial accordance with the teaching of Example 4 except that plasmid pHJL284, rather than plasmid pHJL280, was used. The desired strain was cultured and produced macrocin O-methyltransferase and tylosin at levels above that produced in the untransformed S. fradiae GS15 strain.

Example 6

## Construction of Streptomyces fradiae GS16/pHJL280

The desired strain was constructed in substantial accordance with the teaching of Example 3 except that Streptomyces fradiae GS16, rather than S. fradiae GS28, was used. The desired strain was cultured and produced the tylE gene product, demethylmacrocin O-methyltransferase, and tylosin at levels above that produced in the untransformed strain. The demethylmacrocin O-methyltransferase activity and tylosin production respectively are assayed and determined in substantial accordance with the above-referenced procedures, except that demethylmacrocin is substituted for macrocin as substrate.

Example 7

## Construction of Streptomyces fradiae GS76/pHJL280

The desired strain was constructed in substantial accordance with the teaching of Example 3 except that Streptomyces fradiae GS76, rather than S. fradiae GS28, was used. The desired strain was cultured and produced the tylD and tylH gene products and tylosin at levels above that produced in the untransformed strain.

Example 8

## Construction of Streptomyces fradiae GS48/pHJL280

The desired strain was constructed in substantial accordance with the teaching of Example 3 except that Streptomyces fradiae GS48, rather than S. fradiae GS28, was used. The desired strain was cultured and produced the tylD gene product and tylosin at levels above that produced in the untransformed strain.

Example 9

## Construction of Streptomyces fradiae GS52/pHJL284

The desired strain was constructed in substantial accordance with the teaching of Example 3 except that Streptomyces fradiae GS52 and plasmid pHJL284, rather than S. fradiae GS28 and plasmid pHJL280, were used. The desired strain was cultured and produced the tylC gene product and tylosin at levels above that produced in the untransformed strain.

Example 10

Specific Activity of Rate-Limiting Enzymes and Increased Tylosin Production Using the Present Method

The following Tables demonstrate the effectiveness of the present method. All transformants listed in the Tables were obtained in substantial accordance with the procedure of Example 3. The results indicated in Tables XII and XIII were obtained from strains cultured in fermentation media (Baltz and Seno, 1981, Antimicrobial Agents and Chemotherapy 20:214-225) that also contained 20 $\mu$g/ml thiostrepton if the strain being cultured harbored a plasmid. Note that the transformed strains listed in Tables XII and XIII are low tylosin-producing, or produce amounts of tylosin that are not readily detectable, and were cultured in the presence of selective pressure (thiostrepton) for plasmid maintenance as an autonomously replicating vector.

## Table XII

Specific Activity of the tylF Gene Product, Macrocin O-methyltransferase (MOMT)

| | Strain | Transforming Plasmid | MOMT Specific Activity | | | |
|---|---|---|---|---|---|---|
| | | | 2 days[1] | 3 days | 4 days | 6 days |
| Run 1 | GS15 | pHJL210[4] | 0 | 0 | 0 | 0 |
| | GS15 | pHJL280 | 1.14 | 1.93 | NT | NT |
| | C4[2] | None | Not tested (NT) | 0.35 | 0.16 | NT |
| Run 2 | GS15 | pHJL210 | 0 | 0 | 0 | 0 |
| | GS15 | pHJL280 | 4.2 | 3.2 | 2.2 | 1.8 |
| | C4 | None | 0.8 | 1.0 | 0.9 | 0.9 |
| | T1405[3] | None | 0.9 | 1.2 | 1.5 | 1.4 |
| Run 3 | GS28 | None | 0 | 0.01 | 0.03 | NT |
| | GS28 | pHJL210 | 0 | 0 | 0 | NT |
| | GS28 | pHJL280 | 0.8 | 0.7 | 1.0 | NT |
| | GS28 | pHJL284 | 0.9 | 1.2 | 0.9 | NT |
| | C4 | None | 0.2 | 0.6 | 0.5 | NT |

[1] days in fermentation.

[2] the strain from which GS15 and GS28 were derived.

[3] a strain derived from C4.

[4] the cloning vector into which the tyl genes were inserted to obtain plasmids pHJL280 and pHJL284.

Table XIII

Specific Activity of the tylE Gene Product, Demethylmacrocin O-methyltransferase (DMOMT)

| Strain | Transforming Plasmid | DMOMT Specific Activity | | |
|---|---|---|---|---|
| | | 2 days* | 3 days | 4 days |
| GS16 | pHJL210 | 0 | 0 | 0 |
| GS16 | pHJL280 | 1.8 | 3.7 | 4.0 |
| GS16 | pHJL280 | 3.8 | 1.7 | 3.0 |
| GS16 | pHJL284 | 1.3 | 1.6 | 2.2 |
| C4 | pHJL210 | 0.7 | 1.3 | 1.5 |
| C4 | pHJL210 | 0.2 | 1.1 | 1.9 |
| C4 | None | 0.4 | 1.5 | 1.0 |

The results in Table XIV were obtained from transformants of high tylosin-producing strains that were cultured post-transformation to obtain integrants, transformants in which all or part of the plasmid DNA has integrated into the genome of the host cell. Two methods were used to obtain the integrants. In the first method, transformants are passaged onto selective (contains thiostrepton) and nonselective plates and incubated about 16 hours at 29°C to obtain single colonies. The single colonies on the nonselective plates that were thiostrepton-resistant on the selective plate are repassaged several times in the same manner until a single colony was found to be relatively stable without selection. In the second method for obtaining integrants, the transformants were nonselectively passaged several times by transferring spores from the surface of the plate using a cotton swab. After several passages, the colonies are grown in non-selective, liquid media (TSB), homogenized, fragmented by sonication, diluted, and plated on selective and nonselective media to identify relatively stable integrants. Other methods of obtaining integrants are apparent to those skilled in the art, and the present method is not limited to a particular method of obtaining integrants.

Relatively stable integrants were used to inoculate vegetative medium (complex vegetative medium contains, per liter, 10 g of corn steep liquor, 5 g of yeast extract, 5 g of soybean grits, 3 g of calcium carbonate, and 4.5 g of crude soybean oil, and the pH is adjusted to 7.8 with NaOH. TSB is also a suitable vegetative media) without thiostrepton (no selective pressure), and the vegetative culture was used to inoculate (10% inoculum) the fermentation medium, which also lacked thiostrepton. Fermentations were run at 260 rpm at 29 °C for seven days. The total macrolide content of the fermentation broth was measured by extraction with methanol: CHCl₃, reading the absorbance at 290 nm, and comparing to a standard curve. Tylosin factors were identified by spotting the fermentation broth onto silica-gel-TLC plates and developing the plates with a solvent system of 95:5 ethylacetate:diethylamine. The concentration of individual macrolide components was the total A₂₉₀ times the percentage of each component as determined by HPLC.

### Table XIV

| Strain | % Thiostrepton Resistant | Transforming Plasmid | DMOMT Specific Activity | MOMT Specific Activity | Tylosin* |
|---|---|---|---|---|---|
| C4 | 0 | None | 0.59 | 0.14 | 1 |
| C4 | 9.5 | pHJL280 | Not tested (NT) | NT | 1.10 |
| C4 | 5.9 | pHJL280 | NT | NT | 0.97 |
| T1405 | 0 | None | 1.0 | 0.17 | 1.14 |
| T1405 | 50 | pHJL280 | 0.91 | 0.26 | 1.52 |
| T1405 | 8.7 | pHJL280 | NT | NT | 1.21 |
| T1405 | 11 | pHJL280 | NT | NT | 1.07 |
| T1405 | 6.4 | pHJL280 | NT | NT | 1.21 |
| T1405 | 18 | pHJL280 | 2.5 | 0.43 | 1.60 |
| T1405 | 4.6 | pHJL280 | NT | NT | 0.98 |
| T1405 | 16 | pHJL280 | NT | NT | 1.07 |
| T1405 | 12 | pHJL280 | NT | NT | 1.10 |
| T1405 | 18 | pHJL280 | NT | NT | 1.28 |
| T1405 | 25 | pHJL280 | NT | NT | 1.28 |
| T1405 | 56 | pHJL280 | 0.82 | 0.22 | 1.45 |

*Relative to C4 strain

37

Example 11

Preparation of Tylosin

A sporulated culture of Streptomyces fradiae containing the plasmids provided by the present invention can be produced by growing the organism on a nutrient agar slant having the following composition:

| | |
|---|---|
| Yeast extract | 1.0 g |
| Beef extract | 1.0 g |
| Hydrolyzed casein ("N-Z-Amine-Type A," sold by the Sheffield Chemical Co.) | 2 g |
| Dextrin | 10 g |
| Cobaltous chloride heptahydrate | 20 mg |
| Agar | 20 g |
| Water | 1 l |

The pH of the medium is adjusted to pH 7.3 by the addition of sodium hydroxide.

The slant is inoculated with spores of the desired organism and is incubated for five days at about 30°C. The sporulated culture growth on the slant is covered with water, and the slant is scraped gently to remove the spores to provide an aqueous spore suspension.

1 ml. of the spore suspension is used to inoculate under aseptic conditions a 100 ml. portion of a sterile vegetative culture medium having the following composition:

| | G. |
|---|---|
| Glucose | 15 |
| Soybean meal | 15 |
| Corn steep solids | 15 |
| Sodium chloride | 5 |
| Calcium carbonate | 2 |
| Tap water, added to make a total of volume of 1 l. | |

The inoculated vegetative medium is incubated at about 30°C. for 48 hours, during which time the incubate is shaken at the rate of 114 cycles per minute on a reciprocal shaker having a 2-inch stroke.

5 ml. of the vegetative inoculum are used to inoculate aseptically 100 ml. portions of the following sterilized production medium contained in 500 ml. Erlenmeyer flasks:

| | |
|---|---|
| Soybean meal | 15 g |
| Casein | 1 g |
| Crude glucose syrup | 20 ml |
| Calcium carbonate | 2.5 g |
| Sodium nitrate | 3 g |
| Tap water, added to make a total of volume of 1 l. | |

The inoculated culture then is incubated for 100 hours at about 26-28°C. During the incubation period, the incubate is shaken at 114 revolutions per minute on a reciprocal shaker having a 2-inch stroke. The pH of the starting medium is about pH 6.5, and at the end of the incubation period, the pH of the medium generally increases to about pH 7.5.

The fermented culture broth is filtered to remove the mycelium and other undissolved solids. The filtered broth contains the tylosin.

Example 12

Alternate Preparation of Tylosin

A sporulated culture of the desired transformed microorganism is produced by growing the organism on a nutrient agar slant having the following composition.

| Tomato paste-oatmeal Agar: | G |
|---|---|
| Tomato paste | 20 |
| Pre-cooked oatmeal | 20 |
| Agar | 15 |
| Water, added to make a total volume of 1 l. | |

The slant is inoculated with spores of the organism and the inoculated slant is incubated for 9 days at a temperature of about 30°C. After incubation, the sporulated culture on the slant is covered with water, and the surface of the slant is scraped gently to remove the spores to obtain an agueous spore suspension.

Employing aseptic techniques, one-half of the inoculum obtained from one agar slant is used for inoculating a 500 ml. portion of a sterilized vegetative culture medium having the following composition contained in a 2 l. Erlenmeyer flask:

| Corn-steep yeast I: | G. |
|---|---|
| Glucose | 15 |
| Corn steep solids | 5 |
| Yeast | 5 |
| Calcium carbonate | 5 |
| Water, added to make a total volume of 1 l. | |

The incubation is carried on at 28°C for 48 hours with shaking at 110 cycles per minute on a reciprocal shaker having a 2-inch stroke.

0.25 gal. of the vegetative inoculum from the flask is added aseptically as an inoculum to 250 gal. of the sterile corn steep yeast I medium described above contained in an iron 350 gal fermentor. 0.025 gal. of Antifoam A (an antifoam product sold by The Dow Corning Company) is added to the culture medium to prevent excessive foaming, and additional quantities are added as needed during the fermentation. The inoculated medium is fermented for 24 hours at a temperature of 28°C. During fermentation, the medium is aerated with sterile air at a rate of 27 cubic feet per minute and is agitated with two 16-inch impellers operated at 160 revolutions per minute.

To a 1700 gal. iron fermentor are added 1200 gal. of a medium having the following composition.

| Corn steep soy XII: | Kg. |
|---|---|
| Glucose | 30 |
| Soybean oil meal | 15 |
| Corn steep solids | 5 |
| Crude soybean oil | 10 |
| Calcium carbonate | 2 |
| Sodium chloride | 5 |
| Water, added to make a total volume of 1000 l. | |

The medium is inoculated with 96 gal. of the inoculum grown in the fermentation tank. The fermentation is carried on at 28°C for four days, and any foaming is controlled by the addition as needed of "Larex" No. 1 (an antifoam product sold by Swift & Company). The fermentation medium is aerated by the addition of sterile air at the rate of 128 cubic feet per minute and is agitated with two 24-inch impellers operated at 130 revolutions per minute.

39

600 pounds of "Silflo" (a diatomaceous earth filter aid sold by The Silfo Company) are added to the broth, and the mixture is filtered. The filtrate is adjusted to pH 8.5 by the addition of 20 percent sodium hydroxide, 500 gal. of chloroform are added, the mixture is stirred for 30 minutes, and the chloroform layer which is in the form of an emulsion is decanted. The chloroform extraction is repeated twice with 500-gal. portions of chloroform. The chloroform emulsions which contain the tylosin are combined and are passed through a De Laval separator to break the emulsion, and the chloroform solution is then concentrated in vacuo to a volume of 25 l. The impurities are largely removed from the solution by passing it over a column 6 inches in diameter containing 10 kg. of activated carbon such as that sold by the Pittsburgh Coke and Chemical Co. The carbon column is washed with 16 l. of chloroform, and the combined chloroform effluents containing the tylosin are concentrated in vacuo to a volume of about 2 l. The chloroform concentrate is added slowly with stirring to 20 l. of petroleum ether, the mixture is stirred for 15 minutes, it is filtered to remove the white, amorphous precipitate of tylosin.

The amorphous tylosin is crystallized by dissolving it in 355 ml. of acetone, filtering the acetone mixture to remove a slight haze, and slowly adding the filtered acetone mixture with gentle stirring to 20 l. of water at 5°C. The aqueous, acetone solution of tylosin is permitted to stand at room temperature with gentle stirring to permit the acetone to evaporate slowly, whereupon tylosin crystallizes out. The tylosin crystals are removed by filtration and are dried in vacuo at room temperature. Tylosin has a melting point of about 127-132°C.

## Example 13

### Preparation of Tylosin Tartrate

5 g. of crystalline tylosin are dissolved in 100 ml. of acetone, and 1.5 g. of D-tartaric acid dissolved in 20 ml. of acetone are added with stirring. The solution is permitted to stand at room temperature whereupon the tartrate salt of tylosin crystallizes out of the solution. The crystals of the tartrate salt of tylosin are removed by filtration, are washed with acetone, and are air-dried. The crystalline tartrate salt of tylosin melts at about 140-146°C.

## Example 14

### Preparation of Tylosin Gluconate

1.03 g. glucono-delta lactone are dissolved in 10 ml. of water, and the aqueous solution is warmed to 85°C. for two hours to cause hydrolysis of the lactone to gluconic acid. 15 ml. of warm methanol are added to the aqueous solution. 5 g. of tylosin dissolved in 10 ml. of methanol are added to the methanol mixture with stirring. The tylosin methanol mixture is permitted to stand overnight at room temperature. The methanol is removed from the mixture by evaporation in vacuo at room temperature. After the methanol is removed, 40 ml. of water are added to the aqueous tylosin mixture. The diluted mixture is filtered, and the filtrate containing the tylosin is freeze-dried, producing a white solid consisting of the gluconate salt of tylosin. Tylosin gluconate salt melts at about 114-117°C.

### Example 15

### Preparation of Tylosin Hydrochloride

890 mg. of tylosin are dissolved in 200 ml. of ether. The ether mixture is acidified by the addition of 0.082 ml. of 12 N hydrochloric acid. The precipitate of the hydrochloride salt of tylosin which forms is filtered off, is washed with ether, and is dried in vacuo. The hydrochloride salt of tylosin is recrystallized from an ethanol-ether mixture. The hydrochloride salt of tylosin has melting point of about 141-145°C.

## Claims
### Claims for the following Contracting States : BE, CH, DE, FR, GB, GR, IT, LI, LU, NL, SE

1. A method for increasing the tylosin or tylosin precursor-producing ability of a microorganism, which comprises culturing a microorganism which produces tylosin or a tylosin precursor, said microorganism being transformed with a DNA cloning vector or portion thereof which contains a tylosin or tylosin precursor biosynthetic gene comprising tylC obtainable from plasmid pHJL2804 (Figure 3), NRRL

EP 0 238 323 B1

B-18044, or from plasmid pHJL311 (Figure 5), NRRL B-18046,

tylD obtainable from plasmid pHJL280 (Figure 2), NRRL B-18043, or from plasmid pHJL315 (Figure 6), NRRL B-18047,

tylE obtainable from plasmid pHJL280 (Figure 2), NRRL B-18043, or from plasmid pHJL315 (Figure 6), NRRL B-18047,

tylF obtainable from plasmid pHJL280 (Figure 2), NRRL B-18043, or from plasmid pHJL284 (Figure 3), NRRL B-18044, from plasmid pHJL311 (Figure 5), NRRL B-18046, or from plasmid pHJL315 (Figure 6), NRRL B-18047,

tylH obtaintable from plasmid pHJL280 (Figure 2), NRRL B-18043, from plasmid pHJL311 (Figure 5), NRRL B-18046, or from plasmid pHJL315 (Figure 6), NRRL B-18047,

tylJ obtainable from plasmid pHJL280 (Figure 2), NRRL B-18043, from plasmid pHJL284 (Figure 3), NRRL B-18044, from plasmid pHJL311 (Figure 5), NRRL B-18046, or from plasmid pHJL315 (Figure 6), NRRL B-18047,

tylK obtainable from plasmid pHJL311 (Figure 5), NRRL B-18046,

tylL obtainable from plasmid pHJL309 (Figure 4), NRRL B-18045, or

tylM obtainable from plasmid pHJL309 (Figure 4), NRRL B-18046,

under conditions suitable for cell growth, expression of the tylosin or tylosin precursor gene and production of tylosin or tylosin precursor, provided that the culturing process provides an increase in the tylosin-or tylosin precursor-producing ability of the microorganism.

2. A method as claimed in Claim 1 in which the microorganism is Streptomyces, Cephalosporium, or Penicillium.

3. A method as claimed in Claim 2 in which the microorganism is S. fradiae, S. rimosus, and S. hygroscopicus.

4. A method as claimed in Claim 3 in which the tylosin biosynthetic gene is tylC, tylD, tylE, tylF, tylH, tylJ, tylK, tylL, or tylM.

5. A method as claimed in any one of Claims 1 to 4 in which the biosynthetic gene is tylF.

6. A method as claimed in Claim 1 in which the cloning vector is plasmid pHJL280, pHJL284, pHJL309, pHJL311, or pHJL315.

7. A method as claimed in Claim 1 for producing Streptomyces fradiae GS15/pHJL280 by transforming S. fradia
GS15 (NRRL 18058) with pHJL280 (NRRL B-18043),
S. fradiae GS15/pHJL284 by transforming S. fradiae GS15 (NRRL 18058) with pHJL284 (NRRL B-18044),
S. fradiae GS15/pHJL311 by transforming S. fradiae GS15 (NRRL 18058) with pHJL311 (NRRL B-18046),
S. fradiae GS15/pHJL315 by transforming S. fradiae GS15 (NRRL 18058) with pHJL315 (NRRL B-18047),
S. fradiae GS28/pHJL280 by transforming S. fradiae GS28 (NRRL 18059) with pHJL280 (NRRL B-18043),
S. fradiae GS28/pHJL284 by transforming S. fradiae GS28 (NRRL 18059) with pHJL284 (NRRL B-18044),
S. fradiae GS28/pHJL311 by transforming S. fradiae GS28 (NRRL 18059) with pHJL311 (NRRL B-18046),
S. fradiae GS28/pHJL315 by transforming S. fradiae GS28 (NRRL 18059) with pHJL315 (NRRL B-18047),
S. fradiae GS16/pHJL280 by transforming S. fradiae GS16 with pHJL280 (NRRL B-18043),
S. fradiae GS16/pHJL315 by transforming S. fradiae GS16 with pHJL315 (NRRL B-18047),
S. fradiae GS48/pHJL280 by transforming S. fradiae GS48 with pHJL280 (NRRL B-18043),
S. fradiae GS48/pHJL315 by transforming S. fradiae GS48 with pHJL315 (NRRL B-18047),
S. fradiae GS52/pHJL284 by transforming S. fradiae GS52 (NRRL 18060) with pHJL284 (NRRL B-18044),
S. fradiae GS52/pHJL311 by transforming S. fradiae GS52 (NRRL 18060) with pHJL311 (NRRL B-

41

18046),

S. fradiae GS76/pHJL280 by transforming S. fradiae GS76 with pHJL280 (NRRL B-18043), or
S. fradiae GS76/pHJL315 by transforming S. fradiae GS76 with pHJL315 (NRRL B-18047).

8. A recombinant DNA cloning vector as defined in Claim 1.

9. Plasmids pHJL280 (NRRL B-18043), pHJL284 (NRRL B-18044), pHJL309 (NRRL B-18045), pHJL311 (NRRL B-18046), or pHJL315 (NRRL B-18047).

10. A microorganism that is transformed with a cloning vector as claimed in Claim 8.

11. A microorganism which is transformed with a plasmid of Claim 9.

12. A microorganism of Claim 11 which is E. coli K12 HB101/pHJL280 (NRRL B-18043), E. coli K12 HB101/pHJL284 (NRRL B-18044), E. coli K12 HB101/pHJL309 (NRRL B-18045), E. coli K12 HB101/pHJL311 (NRRL B-18046), or E. coli K12 JM109/pHJL315 (NRRL B-18047).

13. A microorganism as claimed in Claim 11 which is Streptomyces.

14. A microorganism as claimed in Claim 13 which is Streptomyces fradiae.

15. A microorganism as defined in Claim 7 and as follows:
S. fradiae GS15/pHJL280, S. fradiae GS15/pHJL284, S. fradiae GS15/pHJL311, S. fradiae GS15/pHJL315, S. fradiae GS28/pHJL280, S. fradiae GS28/pHJL284, S. fradiae GS28/pHJL311, S. fradiae GS28/pHJL315, S. fradiae GS16/pHJL315, S. fradiae GS48/pHJL280, S. fradiae GS48/pHJL315, S. fradiae GS52/pHJL284, S. fradiae GS52/pHJL311, S. fradiae GS76/pHJL280, or S. fradiae GS76/pHJL315.

16. A DNA sequence that encodes a gene for the tylC, tylD, tylE, tylF, tylH, tylJ, tylK, tylL, or tylM biosynthetic genes, as defined in Claim 1.

17. A process for preparing tylosin, or a pharmaceutically acceptable salt thereof, which comprises culturing a microorganism which produces tylosin or a tylosin precursor by an antibiotic biosynthetic pathway, said microorganism being transformed with a DNA cloning vector, or portion thereof, in a culture medium containing assimilable sources of carbon, nitrogen and inorganic salts under aerobic fermentation conditions characterized in that the DNA cloning vector, or portion thereof, comprises a tylosin or tylosin precursor biosynthetic gene which codes for the expression of a rate-limiting enzyme or gene product of the tylosin or tylosin precursor biosynthetic gene as defined in Claim 1,which codes for the expression of a rate-limiting enzyme or gene product of the tylosin or tylosin precursor biosynthetic pathway, said biosynthetic gene being expressed under fermentation conditions providing for an increase in the tylosin- or tylosin precursor-producing ability of the microorganism.

18. A recombinant DNA sequence comprising the tylF gene as defined in Claim 1.

19. The tylF gene claimed having the sequence:

```
                10               20              30              40
5'-TTC GCG GGA TGG ATG CTG ACC CGG GGG TCG GCC AGC AGC GCC CGG ACG


    50              60              70              80              90
TGA TCT GGC GGG AGA TCA GCC AGA CCG GCG CCC CGT CCC ACA GCT CGG


        100             110             120             130             140
CCC GGG CGA TCG GCT CCT CCG CCC GGA GGG CGG CGT ACT GCT CGG GAG


        150             160             170             180             190
GGC TGA AGG GAC AGG TGC GGG CGA CCG GCC AGG CGA TGC TGC GCC GGC


            200             210             220             230             240
CTG CGG CCC CGT CGG TGT CGT TGG CGC GTG CTG CGG GCA ACA GAA TCC


                250             260             270             280
CCT TTT GTG ACG GGC GGG CGT CCC CGG ACG AGG ACA CGA CTC GCT GCG


    290             300             310             320             330
GCC TCA ACG AAA ACA CCG TGT CCG GTG CCC AGG CCA CGA ACG GTG ACC


        340             350             360             370             380
GGT CTG TGT CAG GTC GCC CGT GGT GAC GGG CTC CGG GGC GGC GGC GCG
```

```
         390              400              410              420              430
    GGC GGC CGA CCT TGA CAT ACC CGC GGC CGG GCT CCT CGT TCC GGC GCG


             440              450              460              470              480
    GCC CGC GCC GAT AGC GTC CGT CCT CAC CGG CTC CGG CGT CCG CGT CCC


                 490              500              510              520
    CGC CGG GAC GTG CCA CCT CTC CCG ACC CCG CGA GCC GAT CGA CCC GCT


     530              540              550              560              570
    ACT GGA GGA CCC GTG GCA CCT TCC CCG GAC CAC GCC CGC GAT CTC TAC
                     VAL ALA PRO SER PRO ASP HIS ALA ARG ASP LEU TYR
                                          5                        10


         580              590              600              610              620
    ATC GAG CTG CTG AAG AAG GTC GTC TCG AAC GTC ATC TAC GAG GAC CCC
    ILE GLU LEU LEU LYS LYS VAL VAL SER ASN VAL ILE TYR GLU ASP PRO
                 15                      20                      25


         630              640              650              660              670
    ACC CAT GTG GCG GGG ATG ATC ACC GAC GCG TCG TTC GAC CGG ACG TCC
    THR HIS VAL ALA GLY MET ILE THR ASP ALA SER PHE ASP ARG THR SER
         30                      35                      40


             680              690              700              710              720
    CGT GAG AGC GGC GAG GAC TAC CCC ACG GTC GCC CAC ACG ATG ATC GGC
    ARG GLU SER GLY GLU ASP TYR PRO THR VAL ALA HIS THR MET ILE GLY
    45                      50                      55                      60
```

44

EP 0 238 323 B1

```
        730             740             750             760
CTC AAG CGT CTG GAC AAT CTC CAC CGG TGC CTC GCG GAC GTC GTG GAG
LEU LYS ARG LEU ASP ASN LEU HIS ARG CYS LEU ALA ASP VAL VAL GLU
                65                      70                      75


  770         780             790             800         810
GAC GGC GTC CCC GGT GAC TTC ATC GAG ACC GGG GTG TGC CGC GCG CCG
ASP GLY VAL PRO GLY ASP PHE ILE GLU THR GLY VAL CYS ARG ALA PRO
                80                      85                      90


    820             830             840             850         860
TGC ATC TTC GCC CGC GGA CTG CTG AAC GCG TAC GGC CAG GCC GAC CGC
CYS ILE PHE ALA ARG GLY LEU LEU ASN ALA TYR GLY GLN ALA ASP ARG
          95                      100                     105


      870             880             890             900         910
ACC GTC TGG GTC GCC GAC TCC TTC CAG GGC TTT CCC GAG CTG ACC GGG
THR VAL TRP VAL ALA ASP SER PHE GLN GLY PHE PRO GLU LEU THR GLY
          110                     115                     120


        920             930             940             950         960
TCC GAC CAC CCG CTG GAC GTC GAG ATC GAC CTC CAC CAG TAC AAC GAG
SER ASP HIS PRO LEU ASP VAL GLU ILE ASP LEU HIS GLN TYR ASN GLU
125                     130                     135                     140


          970             980             990             1000
GCC GTG GAC CTG CCC ACC AGC GAG GAG ACC GTG CGG GAG AAC TTC GCC
ALA VAL ASP LEU PRO THR SER GLU GLU THR VAL ARG GLU ASN PHE ALA
                145                     150                     155
```

45

```
      1010          1020          1030          1040          1050
CGG TAC GGG CTG CTC GAC GAC AAC GTC CGT TTC CTG GCG GGG TGG TTC
ARG TYR GLY LEU LEU ASP ASP ASN VAL ARG PHE LEU ALA GLY TRP PHE
                160                    165                    170


       1060          1070          1080          1090          1100
AAG GAC ACC ATG CCG GCT GCG CCC GTG AAG CAG CTC GCG GTG ATG CGC
LYS ASP THR MET PRO ALA ALA PRO VAL LYS GLN LEU ALA VAL MET ARG
             175                    180                    185


       1110          1120          1130          1140          1150
CTG GAC GGC GAC TCC TAC GGC GCC ACC ATG GAT GTG CTC GAC AGC CTG
LEU ASP GLY ASP SER TYR GLY ALA THR MET ASP VAL LEU ASP SER LEU
      190                    195                    200


         1160          1170          1180          1190          1200
TAC GAG CGG CTG TCG CCG GGC GGT TAC GTC ATC GTC GAC GAC TAC TGC
TYR GLU ARG LEU SER PRO GLY GLY TYR VAL ILE VAL ASP ASP TYR CYS
205                    210                    215                    220


          1210          1220          1230          1240
TC CCG GCC TGC CGC GAG CGG TGC ACG ACT TCC GCG ACC GGC TCG GCA
LE PRO ALA CYS ARG GLU ARG CYS THR THR SER ALA THR GLY SER ALA
                225                    230                    235


   1250          1260          1270          1280          1290
TCC GCG ACA CGA TCC ACC GGA TCG ACC GCC AGG GCG CTA TTG GCG GCA
SER ALA THR ARG SER THR GLY SER THR ALA ARG ALA LEU LEU ALA ALA
             240                    245                    250
```

46

EP 0 238 323 B1

```
        1300            1310           1320            1330           1340
   CAG CGG CTG AGT CGT TCC GCC CGA GAG CCC GAC GAG AGC AGG AGA TAT
   GLN ARG LEU SER ARG SER ALA ARG GLU PRO ASP GLU SER ARG ARG TYR
        255                            260                            265


        1350            1360           1370            1380           1390
   GCG AGA CAC GAC GCG CCC GCT CGG CAT TGA GGG AGC GTG GGT GAT CCA
   ALA ARG HIS ASP ALA PRO ALA ARG HIS
        270                            275


        1400            1410           1420            1430           1440
   GCC GGA GAT CCA TCC GGA CCG GCG CGG CGA GTT CCA CGC GTG GTT CCA


        1450            1460           1470            1480
   GAG CCA GCC GAG TTC CGG CGG CTG ACC GGT CAC TCC TTC TCC GTG CCG


        1490           1500            1510            1520           1530
   CAG GTC GTC AAT ATC GCG TGT CCC GGA AAG GCG CCG CTG CGG CAT CCA


        1540           1550            1560            1570           1580
   CTT CTG CCG AGG TGC CAC CGG GCC GAG GCC AAG TAC AGC GGC GTG TGT


        1590           1600            1610            1620           1630
   GCA GGG CGC CGG TGT CGA GGT CGT CGT CGA CGC GCC GGT GTC GAG GTC


        1640
   GTC GTC GAC-3'
```

wherein A is deoxyadenyl residue; G is a deoxyquanyl residue; C is a deoxycytidyl residue; and T is a deoxythymidyl residue.

20. The amino acid sequence encoded by nucleotides 541 to 1371 of the tylF gene defined in claim 19.

**Claims for the following Contracting States : AT, ES**

1. A method for increasing the tylosin or tylosin precursor-producing ability of a microorganism, which comprises culturing a microorganism which produces tylosin or a tylosin precursor, said microorganism being transformed with a DNA cloning vector or portion thereof which contains a tylosin or tylosin precursor biosynthetic gene comprising tylC obtainable from plasmid pHJL2804 (Figure 3), NRRL B-18044, or from plasmid pHJL311 (Figure 5), NRRL B-18046,

47

tylD obtainable from plasmid pHJL280 (Figure 2), NRRL B-18043, or from plasmid pHJL315 (Figure 6), NRRL B-18047,

tylE obtainable from plasmid pHJL280 (Figure 2), NRRL B-18043, or from plasmid pHJL315 (Figure 6), NRRL B-18047,

tylF obtainable from plasmid pHJL280 (Figure 2), NRRL B-18043, or from plasmid pHJL284 (Figure 3), NRRL B-18044, from plasmid pHJL311 (Figure 5), NRRL B-18046, or from plasmid pHJL315 (Figure 6), NRRL B-18047,

tylH obtaintable from plasmid pHJL280 (Figure 2), NRRL B-18043, from plasmid pHJL311 (Figure 5), NRRL B-18046, or from plasmid pHJL315 (Figure 6), NRRL B-18047,

tylJ obtainable from plasmid pHJL280 (Figure 2), NRRL B-18043, from plasmid pHJL284 (Figure 3), NRRL B-18044, from plasmid pHJL311 (Figure 5), NRRL B-18046, or from plasmid pHJL315 (Figure 6), NRRL B-18047,

tylK obtainable from plasmid pHJL311 (Figure 5), NRRL B-18046,

tylL obtainable from plasmid pHJL309 (Figure 4), NRRL B-18045, or

tylM obtainable from plasmid pHJL309 (Figure 4), NRRL B-18046,

under conditions suitable for cell growth, expression of the tylosin or tylosin precursor gene and production of tylosin or tylosin precursor, provided that the culturing process provides an increase in the tylosin-or tylosin precursor-producing ability of the microorganism.

2. A method as claimed in Claim 1 in which the microorganism is Streptomyces, Cephalosporium, or Penicillium.

3. A method as claimed in Claim 2 in which the microorganism is S. fradiae, S. rimosus, and S. hygroscopicus.

4. A method as claimed in Claim 3 in which the tylosin biosynthetic gene is tylC, tylD, tylE, tylF, tylH, tylJ, tylK, tylL, or tylM.

5. A method as claimed in any one of Claims 1 to 4 in which the biosynthetic gene is tylF.

6. A method as claimed in Claim 1 in which the cloning vector is plasmid pHJL280, pHJL284, pHJL309, pHJL311, or pHJL315.

7. A method as claimed in Claim 1 for producing Streptomyces fradiae GS15/pHJL280 by transforming S. fradia
GS15 (NRRL 18058) with pHJL280 (NRRL B-18043),
S. fradiae GS15/pHJL284 by transforming S. fradiae GS15 (NRRL 18058) with pHJL284 (NRRL B-18044),
S. fradiae GS15/pHJL311 by transforming S. fradiae GS15 (NRRL 18058) with pHJL311 (NRRL B-18046),
S. fradiae GS15/pHJL315 by transforming S. fradiae GS15 (NRRL 18058) with pHJL315 (NRRL B-18047),
S. fradiae GS28/pHJL280 by transforming S. fradiae GS28 (NRRL 18059) with pHJL280 (NRRL B-18043),
S. fradiae GS28/pHJL284 by transforming S.fradiae GS28 (NRRL 18059) with pHJL284 (NRRL B-18044),
S. fradiae GS28/pHJL311 by transforming S. fradiae GS28 (NRRL 18059) with pHJL311 (NRRL B-18046),
S. fradiae GS28/pHJL315 by transforming S. fradiae GS28 (NRRL 18059) with pHJL315 (NRRL B-18047),
S. fradiae GS16/pHJL280 by transforming S. fradiae GS16 with pHJL280 (NRRL B-18043),
S. fradiae GS16/pHJL315 by transforming S. fradiae GS16 with pHJL315 (NRRL B-18047),
S. fradiae GS48/pHJL280 by transforming S. fradiae GS48 with pHJL280 (NRRL B-18043),
S. fradiae GS48/pHJL315 by transforming S. fradiae GS48 with pHJL315 (NRRL B-18047),
S. fradiae GS52/pHJL284 by transforming S. fradiae GS52 (NRRL 18060) with pHJL284 (NRRL B-18044),
S. fradiae GS52/pHJL311 by transforming S. fradiae GS52 (NRRL 18060) with pHJL311 (NRRL B-18046),

48

S. fradiae GS76/pHJL280 by transforming S. fradiae GS76 with pHJL280 (NRRL B-18043), or
S. fradiae GS76/pHJL315 by transforming S. fradiae GS76 with pHJL315 (NRRL B-18047).

8. A process for preparing tylosin, or a pharmaceutically acceptable salt thereof, which comprises culturing a microorganism which produces tylosin or a tylosin precursor by an antibiotic biosynthetic pathway, said microorganism being transformed with a DNA cloning vector, or portion thereof, in a culture medium containing assimilable sources of carbon, nitrogen and inorganic salts under aerobic fermentation conditions characterized in that the DNA cloning vector, or portion thereof, comprises a tylosin or tylosin precursor biosynthetic gene which codes for the expression of a rate-limiting enzyme or gene product of the tylosin or tylosin precursor biosynthetic gene as defined in Claim 1,which codes for the expression of a rate-limiting enzyme or gene product of the tylosin or tylosin precursor biosynthetic pathway, said biosynthetic gene being expressed under fermentation conditions providing for an increase in the tylosin- or tylosin precursor-producing ability of the microorganism.

**Patentansprüche**
**Patentansprüche für folgende Vertragsstaaten : BE, CH, DE, FR, GB, GR, IT, LI, LU, NL, SE**

1. Verfahren zur Steigerung der Fähigkeit eines Mikroorganismus zur Bildung von Tylosin oder eines Tylosinvorläufers, gekennzeichnet durch die Kultivierung eines Mikroorganismus, der Tylosin oder einen Tylosinvorläufer bildet, wobei dieser Mikroorganismus mit einem DNA Klonierungsvektor oder einem Teil hiervon transformiert ist, der ein Tylosin- oder Tylosinvorläuferbiosynthesegen enthält, das umfaßt

   tylC, erhältlich aus Plasmid pHJL284 (Figur 3), NRRL B-18044, oder von Plasmid pHJL311 (Figur 5), NRRL B-18046,

   tylD, erhältlich aus Plasmid pHJL280 (Figur 2), NRRL B-18043, oder von Plasmid pHJL315 (Figur 6), NRRL B-18047,

   tylE, erhältlich aus Plasmid pHJL280 (Figur 2), NRRL B-18043, oder von Plasmid pHJL315 (Figur 6), NRRL B-18047,

   tylF, erhältlich aus Plasmid pHJL280 (Figur 2), NRRL B-18043, oder von Plasmid pHJL284 (Figur 3), NRRL B-18044, von Plasmid pHJL311 (Figur 5), NRRL B-18046, oder von Plasmid pHJL315 (Figur 6), NRRL B-18047,

   tylH, erhältlich aus Plasmid pHJL280 (Figur 2), NRRL B-18043, von Plasmid pHJL311 (Figur 5), NRRL B-18046, oder von Plasmid pHJL315 (Figur 6), NRRL B-18047,

   tylJ, erhältlich aus Plasmid pHJL280 (Figur 2), NRRL B-18043, von Plasmid pHJL284 (Figur 3), NRRL B-18044, von Plasmid pHJL311 (Figur 5), NRRL B-18046, oder von Plasmid pHJL315 (Figur 6), NRRL B-18047,

   tylK, erhältlich aus Plasmid pHJL311 (Figur 5), NRRL B-18046,

   tylL, erhältlich aus Plasmid pHJL309 (Figur 4), NRRL B-18045, oder

   tylM, erhältlich aus Plasmid pHJL309 (Figur 4), NRRL B-18046,

   unter Bedingungen, die für das Zellwachstum, die Expression des Tylosin- oder Tylosinvorläufergens und zur Bildung von Tylosin oder des Tylosinvorläufers geeignet sind, mit der Maßgabe, daß das Kultivierungsverfahren eine Steigerung der Tylosin- oder Tylosinvorläufer-bildenden Fähigkeit des Mikroorganismus liefert.

2. Verfahren nach Anspruch 1, worin der Mikroorganismus Streptomyces, Cephalosporirnm oder Penicillium ist.

3. Verfahren nach Anspruch 2, worin der Mikroorganismus S. fradiae, S. rimosus und S. hygroscopicus ist.

4. Verfahren nach Anspruch 3, worin das Tylosinbiosythesegen tylC, tylD, tylE, tylF, tylH, tylJ, tylK, tylL oder tylM ist.

5. Verfahren nach einem der Ansprüche 1 bis 4, worin das Biosynthesegen tylF ist.

6. Verfahren nach Anspruch 1, worin der Klonierungsvektor das Plasmid pHJL280, pHJL284, pHJL309, pHJL311 oder pHJL315 ist.

7. Verfahren nach Anspruch 1 zur Herstellung von

Streptomyces fradiae GS15/pHJL280 durch Transformation von S. fradiae GS15 (NRRL 18058) mit pHJL280 (NRRL B-18043),

S. fradiae GS15/pHJL284 durch Transformation von S. fradiae GS15 (NRRL 18058) mit pHJL284 (NRRL B-18044),

S. fradiae GS15/pHJL311 durch Transformation von S. fradiae GS15 (NRRL 18058) mit pHJL311 (NRRL B-18046),

S. fradiae GS15/pHJL315 durch Transformation von S. fradiae GS15 (NRRL 18058) mit pHJL315 (NRRL B-18047),

S. fradiae GS28/pHJL280 durch Transformation von S. fradiae GS28 (NRRL 18059) mit pHJL280 (NRRL B-18043),

S. fradiae GS28/pHJL284 durch Transformation von S. fradiae GS28 (NRRL 18059) mit pHJL284 (NRRL B-18044),

S. fradiae GS28/pHJL311 durch Transformation von S. fradiae GS28 (NRRL 18059) mit pHJL311 (NRRL B-18046),

S. fradiae GS28/pHJL315 durch Transformation von S. fradiae GS28 (NRRL 18059) mit pHJL315 (NRRL B-18047),

S. fradiae GS16/pHJL280 durch Transformation von S. fradiae GS16 mit pHJL280 (NRRL B-18043),

S. fradiae GS16/pHJL315 durch Transformation von S. fradiae GS16 mit pHJL315 (NRRL B-18047),

S. fradiae GS48/pHJL280 durch Transformation von S. fradiae GS48 mit pHJL280 (NRRL B-18043),

S. fradiae GS48/pHJL315 durch Transformation von S. fradiae GS48 mit pHJL315 (NRRL B-18047),

S. fradiae GS52/pHJL284 durch Transformation von S. fradiae GS52 (NRRL 18060) mit pHJL284 (NRRL B-18044),

S. fradiae GS52/pHJL311 durch Transformation von S. fradiae GS52 (NRRL 18060) mit pHJL311 (NRRL B-18046),

S. fradiae GS76/pHJL280 durch Transformation von S. fradiae GS76 mit pHJL280 (NRRL B-18043), oder

S. fradiae GS76/pHJL315 durch Transformation von S. fradiae GS76 (NRRL 18058) mit pHJL315 (NRRL B-18047).

8. Rekombinanter DNA Klonierungsvektor nach Anspruch 1.

9. Plasmide pHJL280 (NRRL B-18043, pHJL284 (NRRL B-18044), pHJL309 (NRRL B-18045), pHJL311 (NRRL B-18046) oder pHJL315 (NRRL B-18047).

10. Mikroorganismus, der mit einem Klonierungsvektor nach Anspruch 8 transformiert ist.

11. Mikroorganismus, der mit einem Plasmid nach Anspruch 9 transformiert ist.

12. Mikroorganismus nach Anspruch 11, der ist E. coli K12 HB101/pHJL280 (NRRL B-18043), E. coli K12 HB101/pHJL284 (NRRL B-18044), E. coli K12 HB101/pHJL309 (NRRL B-18045), E. coli K12 HB101/pHJL311 (NRRL B-18046) oder E. coli K12 JM109/pHJL315 (NRRL B-18047).

13. Mikroorganismus nach Anspruch 11, der Streptomyces ist.

14. Mikroorganismus nach Anspruch 13, der Streptomyces fradiae ist.

15. Mikroorganismus nach Anspruch 7 und folgende

S. fradiae GS15/pHJL280, S. fradiae GS15/pHJL284, S. fradiae GS15/pHJL311, S. fradiae GS15/pHJL315, S. fradiae GS28/pHJL280, S. fradiae GS28/pHJL284, S. fradiae GS28/pHJL311, S. fradiae GS28/pHJL315, S. fradiae GS16/pHJL315, S. fradiae GS48/pHJL280, S. fradiae GS48/pHJL315, S. fradiae GS52/pHJL284, S. fradiae GS52/pHJL311, S. fradiae GS76/pHJL280, oder S. fradiae GS76/pHJL315.

16. DNA Sequenz, die ein Gen für die tylC-, tylD-, tylE-, tylF-, tylH-, tylJ-, tylK-, tylL- oder tylM-Biosynthesegene nach Anspruch 1 kodiert.

17. Verfahren zur Herstellung von Tylosin oder eines pharmazeutisch annehmbaren Salzes hiervon, gekennzeichnet durch Kultivierung eines Mikroorganismus, der Tylosin oder einen Tylosinvorläufer durch einen Antibiotikumbiosyntheseweg bildet, wobei dieser Mikroorganismus mit einem DNA Klonierungsvektor oder einem Teil hiervon transformiert ist, in einem Kulturmedium, das assimilierbare Quellen von Kohlenstoff, Stickstoff und anorganischen Salzen enthält, unter aeroben Fermentationsbedingungen, dadurch gekennzeichnet, daß der DNA Klonierungsvektor oder ein Teil hiervon ein Tylosin- oder Tylosinvorläuferbiosynthesegen enthält, das für die Expression eines geschwindigkeitslimitierenden Enzyms oder Genprodukts des Tylosin- oder Tylosinvorläuferbiosynthesegens nach Anspruch 1 kodiert, das für die Expression eines geschwindigkeitslimitierenden Enzyms oder Genprodukts des Tylosin- oder Tylosinvorläuferbiosyntheseweges kodiert, wobei das Biosynthesegen unter den Fermentationsbedingungen exprimiert wird und für eine Steigerung der Tylosin- oder Tylosinvorläufer-bildenden Fähigkeit des Mikroorganismus sorgt.

18. Rekombinante DNA Sequenz, die das tylF Gen nach Anspruch 1 umfaßt.

19. tylF Gen mit folgender Sequenz

```
            10          20          30          40
5'-TTC GCG GGA TGG ATG CTG ACC CGG GGG TCG GCC AGC AGC GCC CGG ACG

    50          60          70          80          90
  TGA TCT GGC GGG AGA TCA GCC AGA CCG GCG CCC CGT CCC ACA GCT CGG

    100         110         120         130         140
  CCC GGG CGA TCG GCT CCT CCG CCC GGA GGG CGG CGT ACT GCT CGG GAG

    150         160         170         180         190
  GGC TGA AGG GAC AGG TGC GGG CGA CCG GCC AGG CGA TGC TGC GCC GGC

    200         210         220         230         240
  CTG CGG CCC CGT CGG TGT CGT TGG CGC GTG CTG CGG GCA ACA GAA TCC

    250         260         270         280
  CCT TTT GTG ACG GGC GGG CGT CCC CGG ACG AGG ACA CGA CTC GCT GCG

    290         300         310         320         330
  GCC TCA ACG AAA ACA CCG TGT CCG GTG CCC AGG CCA CGA ACG GTG ACC'

    340         350         360         370         380
  GGT CTG TGT CAG GTC GCC CGT GGT GAC GGG CTC CGG GGC GGC GGC GCG
```

```
        390             400             410             420             430
GGC GGC CGA CCT TGA CAT ACC CGC GGC CGG GCT CCT CGT TCC GGC GCG


        440             450             460             470             480
GCC CGC GCC GAT AGC GTC CGT CCT CAC CGG CTC CGG CGT CCG CGT CCC


        490             500             510             520
CGC CGG GAC GTG CCA CCT CTC CCG ACC CCG CGA GCC GAT CGA CCC GCT


530             540             550             560             570
ACT GGA GGA CCC GTG GCA CCT TCC CCG GAC CAC GCC CGC GAT CTC TAC
                    VAL ALA PRO SER PRO ASP HIS ALA ARG ASP LEU TYR
                                         5                       10


        580             590             600             610             620
ATC GAG CTG CTG AAG AAG GTC GTC TCG AAC GTC ATC TAC GAG GAC CCC
ILE GLU LEU LEU LYS LYS VAL VAL SER ASN VAL ILE TYR GLU ASP PRO
        15                      20                      25


        630             640             650             660             670
ACC CAT GTG GCG GGG ATG ATC ACC GAC GCG TCG TTC GAC CGG ACG TCC
THR HIS VAL ALA GLY MET ILE THR ASP ALA SER PHE ASP ARG THR SER
        30                      35                      40


        680             690             700             710             720
CGT GAG AGC GGC GAG GAC TAC CCC ACG GTC GCC CAC ACG ATG ATC GGC
ARG GLU SER GLY GLU ASP TYR PRO THR VAL ALA HIS THR MET ILE GLY
45                      50                      55                      60
```

```
        730              740              750              760
CTC AAG CGT CTG GAC AAT CTC CAC CGG TGC CTC GCG GAC GTC GTG GAG
LEU LYS ARG LEU ASP ASN LEU HIS ARG CYS LEU ALA ASP VAL VAL GLU
                 65               70                      75


  770            780            790            800            810
GAC GGC GTC CCC GGT GAC TTC ATC GAG ACC GGG GTG TGC CGC GCG CCG
ASP GLY VAL PRO GLY ASP PHE ILE GLU THR GLY VAL CYS ARG ALA PRO
              80               85                      90


    820            830            840            850            860
TGC ATC TTC GCC CGC GGA CTG CTG AAC GCG TAC GGC CAG GCC GAC CGC
CYS ILE PHE ALA ARG GLY LEU LEU ASN ALA TYR GLY GLN ALA ASP ARG
           95               100                     105


      870            880            890            900            910
ACC GTC TGG GTC GCC GAC TCC TTC CAG GGC TTT CCC GAG CTG ACC GGG
THR VAL TRP VAL ALA ASP SER PHE GLN GLY PHE PRO GLU LEU THR GLY
      110                  115                    120


        920            930            940            950            960
TCC GAC CAC CCG CTG GAC GTC GAG ATC GAC CTC CAC CAG TAC AAC GAG
SER ASP HIS PRO LEU ASP VAL GLU ILE ASP LEU HIS GLN TYR ASN GLU
125                  130                    135                    140


          970            980            990            1000
GCC GTG GAC CTG CCC ACC AGC GAG GAG ACC GTG CGG GAG AAC TTC GCC
ALA VAL ASP LEU PRO THR SER GLU GLU THR VAL ARG GLU ASN PHE ALA
                 145                  150                    155
```

```
      1010          1020          1030          1040          1050
CGG TAC GGG CTG CTC GAC GAC AAC GTC CGT TTC CTG GCG GGG TGG TTC
ARG TYR GLY LEU LEU ASP ASP ASN VAL ARG PHE LEU ALA GLY TRP PHE
                    160           165                 170


      1060          1070          1080          1090          1100
AAG GAC ACC ATG CCG GCT GCG CCC GTG AAG CAG CTC GCG GTG ATG CGC
LYS ASP THR MET PRO ALA ALA PRO VAL LYS GLN LEU ALA VAL MET ARG
          175               180               185


      1110          1120          1130          1140          1150
CTG GAC GGC GAC TCC TAC GGC GCC ACC ATG GAT GTG CTC GAC AGC CTG
LEU ASP GLY ASP SER TYR GLY ALA THR MET ASP VAL LEU ASP SER LEU
          190               195               200


      1160          1170          1180          1190          1200
TAC GAG CGG CTG TCG CCG GGC GGT TAC GTC ATC GTC GAC GAC TAC TGC
TYR GLU ARG LEU SER PRO GLY GLY TYR VAL ILE VAL ASP ASP TYR CYS
205                 210               215                 220


      1210          1220          1230          1240
TC CCG GCC TGC CGC GAG CGG TGC ACG ACT TCC GCG ACC GGC TCG GCA
LE PRO ALA CYS ARG GLU ARG CYS THR THR SER ALA THR GLY SER ALA
                    225               230               235


  1250          1260          1270          1280          1290
TCC GCG ACA CGA TCC ACC GGA TCG ACC GCC AGG GCG CTA TTG GCG GCA
SER ALA THR ARG SER THR GLY SER THR ALA ARG ALA LEU LEU ALA ALA
              240               245               250
```

EP 0 238 323 B1

```
        1300            1310            1320            1330            1340
CAG CGG CTG AGT CGT TCC GCC CCA GAG CCC GAC GAG AGC AGG AGA TAT
GLN ARG LEU SER ARG SER ALA ARG GLU PRO ASP GLU SER ARG ARG TYR
        255                             260                             265


        1350            1360            1370            1380            1390
GCG AGA CAC GAC GCG CCC GCT CGG CAT TGA GGG AGC GTG GGT GAT CCA
ALA ARG HIS ASP ALA PRO ALA ARG HIS
        270                             275


        1400            1410            1420            1430            1440
GCC GGA GAT CCA TCC GGA CCG GCG CGG CGA GTT CCA CGC GTG GTT CCA


        1450            1460            1470            1480
GAG CCA GCC GAG TTC CGG CGG CTG ACC GGT CAC TCC TTC TCC GTG CCG


1490            1500            1510            1520            1530
CAG GTC GTC AAT ATC GCG TGT CCC GGA AAG GCG CCG CTG CGG CAT CCA


1540            1550            1560            1570            1580
CTT CTG CCG AGG TGC CAC CGG GCC GAG GCC AAG TAC AGC GGC GTG TGT


        1590            1600            1610            1620            1630
GCA GGG CGC CGG TGT CGA GGT CGT CGT CGA CGC GCC GGT GTC GAG GTC


        1640
GTC GTC GAC-3'
```

worin A ein Desoxyadenylrest ist, G ein Desoxyguanylrest ist, C ein Desoxycytidylrest ist und T ein Desoxythymidylrest ist.

20. Aminosäuresequenz, die von den Nukleotiden 541 bis 1371 des tyIF Gens nach Anspruch 19 kodiert ist.

**Patentansprüche für folgende Vertragsstaaten : AT, ES**

1. Verfahren zur Steigerung der Fähigkeit eines Mikroorganismus zur Bildung von Tylosin oder eines Tylosinvorläufers, gekennzeichnet durch die Kultivierung eines Mikroorganismus, der Tylosin oder einen Tylosinvorläufer bildet, wobei dieser Mikroorganismus mit einem DNA Klonierungsvektor oder einem Teil hiervon transformiert ist, der ein Tylosin- oder Tylosinvorläuferbiosynthesegen enthält, das umfaßt

tylC, erhältlich aus Plasmid pHJL284 (Figur 3), NRRL B-18044, oder von Plasmid pHJL311 (Figur 5), NRRL B-18046,

tylD, erhältlich aus Plasmid pHJL280 (Figur 2), NRRL B-18043, oder von Plasmid pHJL315 (Figur 6), NRRL B-18047,

tylE, erhältlich aus Plasmid pHJL280 (Figur 2), NRRL B-18043, oder von Plasmid pHJL315 (Figur 6), NRRL B-18047,

tylF, erhältlich aus Plasmid pHJL280 (Figur 2), NRRL B-18043, oder von Plasmid pHJL284 (Figur 3), NRRL B-18044, von Plasmid pHJL311 (Figur 5), NRRL B-18046, oder von Plasmid pHJL315 (Figur 6), NRRL B-18047,

tylH, erhältlich aus Plasmid pHJL280 (Figur 2), NRRL B-18043, von Plasmid pHJL311 (Figur 5), NRRL B-18046, oder von Plasmid pHJL315 (Figur 6), NRRL B-18047,

tylJ, erhältlich aus Plasmid pHJL280 (Figur 2), NRRL B-18043, von Plasmid pHJL284 (Figur 3), NRRL B-18044, von Plasmid pHJL311 (Figur 5), NRRL B-18046, oder von Plasmid pHJL315 (Figur 6), NRRL B-18047,

tylK, erhältlich aus Plasmid pHJL311 (Figur 5), NRRL B-18046,

tylL, erhältlich aus Plasmid pHJL309 (Figur 4), NRRL B-18045, oder

tylM, erhältlich aus Plasmid pHJL309 (Figur 4), NRRL B-18046,

unter Bedingungen, die für das Zellwachstum, die Expression des Tylosin- oder Tylosinvorläufergens und zur Bildung von Tylosin oder des Tylosinvorläufers geeignet sind, mit der Maßgabe, daß das Kultivierungsverfahren eine Steigerung der Tylosin- oder Tylosinvorläufer-bildenden Fähigkeit des Mikroorganismus liefert.

2. Verfahren nach Anspruch 1, worin der Mikroorganismus Streptomyces, Cephalosporium oder Penicillium ist.

3. Verfahren nach Anspruch 2, worin der Mikroorganismus S. fradiae, S. rimosus und S. hygroscopicus ist.

4. Verfahren nach Anspruch 3, worin das Tylosinbiosynthesegen tylC, tylD, tylE, tylF, tylH, tylJ, tylK, tylL oder tylM ist.

5. Verfahren nach einem der Ansprüche 1 bis 4, worin das Biosynthesegen tylF ist.

6. Verfahren nach Anspruch 1, worin der Klonierungsvektor das plasmid pHJL280, pHJL284, pHJL309, pHJL311 oder pHJL315 ist.

7. Verfahren nach Anspruch 1 zur Herstellung von

Streptomyces fradiae GS15/pHJL280 durch Transformation von S. fradiae GS15 (NRRL 18058) mit pHJL280 (NRRL B-18043),

S. fradiae GS15/pHJL284 durch Transformation von S. fradiae GS15 (NRRL 18058) mit pHJL284 (NRRL B-18044),

S. fradiae GS15/pHJL311 durch Transformation von S. fradiae GS15 (NRRL 18058) mit pHJL311 (NRRL B-18046),

S. fradiae GS15/pHJL315 durch Transformation von S. fradiae GS15 (NRRL 18058) mit pHJL315 (NRRL B-18047),

S. fradiae GS28/pHJL280 durch Transformation von S. fradiae GS28 (NRRL 18059) mit pHJL280 (NRRL B-18043),

S. fradiae GS28/pHJL284 durch Transformation von S. fradiae GS28 (NRRL 18059) mit pHJL284 (NRRL B-18044),

S. fradiae GS28/pHJL311 durch Transformation von S. fradiae GS28 (NRRL 18059) mit pHJL311 (NRRL B-18046),

S. fradiae GS28/pHJL315 durch Transformation von S. fradiae GS28 (NRRL 18059) mit pHJL315 (NRRL B-18047),

S. fradiae GS16/pHJL280 durch Transformation von S. fradiae GS16 mit pHJL280 (NRRL B-18043),

S. fradiae GS16/pHJL315 durch Transformation von S. fradiae GS16 mit pHJL315 (NRRL B-18047),

S. fradiae GS48/pHJL280 durch Transformation von S. fradiae GS48 mit pHJL280 (NRRL B-18043),

S. fradiae GS48/pHJL315 durch Transformation von S. fradiae GS48 mit pHJL315 (NRRL B-18047),

S. fradiae GS52/pHJL284 durch Transformation von S. fradiae GS52 (NRRL 18060) mit pHJL284 (NRRL B-18044),

S. fradiae GS52/pHJL311 durch Transformation von S. fradiae GS52 (NRRL 18060) mit pHJL311 (NRRL B-18046),

S. fradiae GS76/pHJL280 durch Transformation von S. fradiae GS76 mit pHJL280 (NRRL B-18043), oder

S. fradiae GS76/pHJL315 durch Transformation von S. fradiae GS76 mit pHJL315 (NRRL B-18047).

8. Verfahren zur Herstellung von Tylosin oder eines pharmazeutisch annehmbaren Salzes hiervon, gekennzeichnet durch Kultivierung eines Mikroorganismus, der Tylosin oder einen Tylosinvorläufer durch einen Antibiotikumbiosyntheseweg bildet, wobei dieser Mikroorganismus mit einem DNA Klonierungsvektor oder einem Teil hiervon transformiert ist, in einem Kulturmedium, das assimilierbare Quellen von Kohlenstoff, Stickstoff und anorganischen Salzen enthält, unter aeroben Fermentationsbedingungen, dadurch gekennzeichnet, daß der DNA Klonierungsvektor oder ein Teil hiervon ein Tylosin- oder Tylosinvorläuferbiosynthesegen enthalt, das für die Expression eines geschwindigkeitslimitierenden Enzyms oder Genprodukts des Tylosin- oder Tylosinvorläuferbiosynthesegens nach Anspruch 1 kodiert, das für die Expression eines geschwindigkeitslimitierenden Enzyms oder Genprodukts des Tylosin- oder Tylosinvorläuferbiosynthesewegs kodiert, wobei das Biosynthesegen unter den Fermentationsbedingungen exprimiert wird und für eine Steigerung der Tylosin- oder Tylosinvorläufer-bildenden Fähigkeit des Mikroorganismus sorgt.

## Revendications
### Revendications pour les Etats contractants suivants : BE, CH, DE, FR, GB, GR, IT, LI, LU, NL, SE

1. Méthode pour accroître la capacité d'un micro-organisme de produire la tylosine ou un précurseur de la tylosine, qui comprend la culture d'un micro-organisme qui produit la tylosine ou un précurseur de la tylosine, ledit micro-organisme étant transformé avec un vecteur de clonage d'ADN ou une traction de celui-ci qui contient un gène biosynthétique de la tylosine ou d'un précurseur de la tylosine comprenant

tylC disponible à partir du plasmide pHJL2804 (Figure 3), NRRL B-18044, ou à partir du plasmide pHJL311 (Figure 5), NRRL B-18046,

tylD disponible à partir du plasmide pHJL280 (Figure 2), NRRL B-18043, ou à partir du plasmide pHJL315 (Figure 6), NRRL B-18047,

tylE disponible à partir du plasmide pHJL280 (Figure 2), NRRL B-18043, ou à partir du plasmide pHJL315 (Figure 6), NRRL B-18047,

tylF disponible à partir du plasmide pHJL280 (Figure 2), NRRL B-18043, ou à partir du plasmide pHJL284 (Figure 3), NRRL B-18044, à partir du plasmide pHJL311 (Figure 5), NRRL B-18046, ou à partir du plasmide pHJL315 (Figure 6), NRRL B-18047,

tylH disponible à partir du plasmide pHJL280 (Figure 2), NRRL B-18043, à partir du plasmide pHJL311 (Figure 5), NRRL B-18046, ou à partir du plasmide pHJL315 (Figure 6), NRRL B-18047,

tylJ disponible à partir du plasmide pHJL280 (Figure 2), NRRL B-18043, à partir du plasmide pHJL284 (Figure 3), NRRL B-18044, à partir du plasmide pHJL311 (Figure 5), NRRL B-18046, ou à partir du plasmide pHJL315 (Figure 6), NRRL B-18047,

tylK disponible à partir du plasmide pHJL311 (Figure 5), NRRL B-18046,

tylL disponible à partir du plasmide pHJL309 (Figure 4), NRRL B-18045, ou

tylM disponible à partir du plasmide pHJL309 (Figure 4), NRRL B-18046,

dans des conditions appropriées à la croissance cellulaire, à l'expression du gène de la tylosine ou d'un précurseur de la tylosine et à la production de la tylosine ou d'un précurseur de la tylosine, pour autant que le procédé de culture fournit un accroissement de la capacité du micro-organisme de produire la tylosine ou un précurseur de la tylosine.

2. Méthode selon la revendication 1, dans laquelle le micro-organisme est Streptomyces, Cephalosporium ou Penicillium.

3. Méthode selon la revendication 2, dans laquelle le micro-organisme est S. fradiae, S. rimosus et S. hygroscopicus.

4. Méthode selon la revendication 3, dans laquelle le gène biosynthétique de la tylosine est tylC, tylD, tylE, tylF, tylH, tylJ, tylK, tylL ou tylM.

**5.** Méthode selon l'une quelconque des revendications 1 à 4, dans laquelle le gène biosynthétique de la tylosine est tylF.

**6.** Méthode selon la revendication 1, dans laquelle le vecteur de clonage est le plasmide pHJL280, pHJL284, pHJL309, pHJL311 ou pHJL315.

**7.** Méthode selon la revendication 1 pour la production de Streptomyces fradiae GS15/pHJL280 en transformant S. fradiae GS15 (NRRL 18058) avec le plasmide pHJL280 (NRRL B-18043),
S. fradiae GS15/pHJL284 en transformant S. fradiae GS15 (NRRL 18058) avec le plasmide pHJL284 (NRRL B-18044),
S. fradiae GS15/pHJL311 en transformant S. fradiae GS15 (NRRL 18058) avec le plasmide pHJL311 (NRRL B-18046),
S. fradiae GS15/pHJL315 en transformant S. fradiae GS15 (NRRL 18058) avec le plasmide pHJL315 (NRRL B-18047),
S. fradiae GS28/pHJL280 en transformant S. fradiae GS28 (NRRL 18059) avec le plasmide pHJL280 (NRRL B-18043),
S. fradiae GS28/pHJL284 en transformant S. fradiae GS28 (NRRL 18059) avec le plasmide pHJL284 (NRRL B-18044),
S. fradiae GS28/pHJL311 en transformant S. fradiae GS28 (NRRL 18059) avec le plasmide pHJL311 (NRRL B-18046),
S. fradiae GS28/pHJL315 en transformant S. fradiae GS28 (NRRL 18059) avec le plasmide pHJL315 (NRRL B-18047),
S. fradiae GS16/pHJL280 en transformant S. fradiae GS16 avec le plasmide pHJL280 (NRRL B-18043),
S. fradiae GS16/pHJL315 en transformant S. fradiae GS16 avec le plasmide pHJL315 (NRRL B-18047),
S. fradiae GS48/pHJL280 en transformant S. fradiae GS48 avec le plasmide pHJL280 (NRRL B-18043),
S. fradiae GS48/pHJL315 en transformant S. fradiae GS48 avec le plasmide pHJL315 (NRRL B-18047),
S. fradiae GS52/pHJL284 en transformant S. fradiae GS52 (NRRL 18060) avec le plasmide pHJL284 (NRRL B-18044),
S. fradiae GS52/pHJL311 en transformant S. fradiae GS52 (NRRL 18060) avec le plasmide pHJL311 (NRRL B-18046),
S. fradiae GS76/pHJL280 en transformant S. fradiae GS76 avec le plasmide pHJL280 (NRRL B-18043), ou
S. fradiae GS76/pHJL315 en transformant S. fradiae GS76 avec le plasmide pHJL315 (NRRL B-18047).

**8.** Vecteur de clonage d'ADN recombinant, tel que défini à la revendication 1.

**9.** Plasmides pHJL280 (NRRL B-18043), pHJL284 (NRRL B-18044), pHJL309 (NRRL B-18045), pHJL311 (NRRL B-18046) ou pHJL315 (NRRL B-18047).

**10.** Micro-organisme qui est transformé avec un vecteur de clonage tel que défini à la revendication 8.

**11.** Micro-organisme qui est transformé avec un plasmide de la revendication 9.

**12.** Micro-organisme de la revendication 11, qui est E. coli K12 HB101/pHJL280 (NRRL B-18043), E. coli K12 HB101/pHJL284 (NRRL B-18044), E. coli K12 HB101/pHJL309 (NRRL B-18045), E. coli K12 HB101/pHJL311 (NRRL B-18046) ou E. coli K12 JM109/pHJL315 (NRRL B-18047).

**13.** Micro-organisme selon la revendication 11, qui est Streptomyces.

**14.** Micro-organisme selon la revendication 13, qui est Streptomyces fradiae.

**15.** Microorganisme tel que défini à la revendication 7 et ci-après :
S. fradiae GS15/pHJL280, S. fradiae GS15/pHJL284,
S. fradiae GS15/pHJL311, S. fradiae GS15/pHJL315,
S. fradiae GS28/pHJL280, S. fradiae GS28/pHJL284,
S. fradiae GS28/pHJL311, S. fradiae GS28/pHJL315,
S. fradiae GS16/pHJL315, S. fradiae GS48/pHJL280,
S. fradiae GS48/pHJL315, S. fradiae GS52/pHJL284,

S. fradiae GS52/pHJL311, S. fradiae GS76/pHJL280,
ou S. fradiae GS76/pHJL315.

16. Séquence d'ADN qui code pour un gène pour les gènes biosynthétiques tylC, tylD, tylE, tylF, tylH, tylJ, tylK, tylL ou tylM, tels que définis à la revendication 1.

17. Procédé pour la préparation de la tylosine, ou d'un sel pharmaceutiquement acceptable de celle-ci, qui comprend la culture d'un micro-organisme qui produit la tylosine ou un précurseur de la tylosine par une voie biosynthétique d'antibiotique, ledit micro-organisme étant transformé avec un vecteur de clonage d'ADN, ou par une fraction de celui-ci, dans un milieu de culture contenant des sources assimilables de carbone, d'azote et de sels inorganiques dans des conditions de fermentation aérobies, caractérisé en ce que le vecteur de clonage d'ADN, ou une fraction de celui-ci, comprend un gène biosynthétique de la tylosine ou d'un précurseur de la tylosine, qui code pour l'expression d'une enzyme ou d'un produit de gène limitatif de la vitesse du gène biosynthétique de la tylosine ou d'un précurseur de la tylosine tel que défini à la revendication 1, qui code pour l'expression d'une enzyme ou d'un produit de gène limitatif de la vitesse de la voie biosynthétique de la tylosine ou d'un précurseur de la tylosine, ledit gène biosynthétique étant exprimé dans des conditions de fermentation permettant un accroissement de la capacité du micro-organisme de produire la tylosine ou un précurseur de la tylosine.

18. Séquence d'ADN recombinant comprenant le gène tylF tel que défini à la revendication 1.

19. Gène tylF revendique présentant la séquence suivante :

EP 0 238 323 B1

```
             10              20              30              40
5'-TTC GCG CGA TGG ATG CTG ACC CGG GGG TCG GCC AGC AGC GCC CGG ACG

     50          60              70          80          90
  TGA TCT GGC GGG AGA TCA GCC AGA CCG GCG CCC CGT CCC ACA GCT CGG

     100         110             120         130         140
  CCC CGG CGA TCG GCT CCT CCG CCC CGA GGG CGG CGT ACT GCT CGG GAG

     150         160             170         180         190
  GGC TGA AGG GAC AGG TGC GGG CGA CCG GCC AGG CGA TGC TGC GCC GGC

         200         210             220         230         240
  CTG CGG CCC CGT CGG TGT CGT TGG CGC GTG CTG CGG GCA ACA GAA TCC

         250         260         270         280
  CCT TTT GTG ACG GGC GGG CGT CCC CGG ACG AGG ACA CGA CTC GCT GCG

     290         300             310         320         330
  GCC TCA ACG AAA ACA CCG TGT CCG GTG CCC AGG CCA CGA ACG GTG ACC

     340         350             360         370         380
  GGT CTG TGT CAG GTC GCC CGT GGT GAC GGG CTC CGG GGC GGC GGC GCG
```

60

```
        390              400              410              420              430
GGC GGC CGA CCT TGA CAT ACC CGC GGC CGG GCT CCT CGT TCC GGC GCG


        440              450              460              470              480
GCC CGC GCC GAT AGC GTC CGT CCT CAC CGG CTC CGG CGT CCG CGT CCC


        490              500              510              520
CGC CGG GAC GTG CCA CCT CTC CCG ACC CCG CGA GCC GAT CGA CCC GCT


530              540              550              560              570
ACT GGA GGA CCC GTG GCA CCT TCC CCG GAC CAC GCC CGC GAT CTC TAC
                VAL ALA PRO SER PRO ASP HIS ALA ARG ASP LEU TYR
                                      5                       10


        580              590              600              610              620
ATC GAG CTG CTG AAG AAG GTC GTC TCG AAC GTC ATC TAC GAG GAC CCC
ILE GLU LEU LEU LYS LYS VAL VAL SER ASN VAL ILE TYR GLU ASP PRO
          15                       20                       25


        630              640              650              660              670
ACC CAT GTG GCG GGG ATG ATC ACC GAC GCG TCG TTC GAC CGG ACG TCC
THR HIS VAL ALA GLY MET ILE THR ASP ALA SER PHE ASP ARG THR SER
          30                       35                       40


        680              690              700              710              720
CGT GAG AGC GGC GAG GAC TAC CCC ACG GTC GCC CAC ACG ATG ATC GGC
ARG GLU SER GLY GLU ASP TYR PRO THR VAL ALA HIS THR MET ILE GLY
45                       50                       55                       60
```

```
          730              740             750              760
CTC AAG CGT CTG GAC AAT CTC CAC CGG TGC CTC GCG GAC GTC GTG GAG
LEU LYS ARG LEU ASP ASN LEU HIS ARG CYS LEU ALA ASP VAL VAL GLU
              65                       70                      75


    770             780             790             800             810
GAC GGC GTC CCC GGT GAC TTC ATC GAG ACC GGG GTG TGC CGC GCG CCG
ASP GLY VAL PRO GLY ASP PHE ILE GLU THR GLY VAL CYS ARG ALA PRO
              80                      85                      90


     820             830             840             850             860
TGC ATC TTC GCC CGC GCA CTG CTG AAC GCG TAC GGC CAG GCC GAC CGC
CYS ILE PHE ALA ARG GLY LEU LEU ASN ALA TYR GLY GLN ALA ASP ARG
              95                      100                     105


      870             880             890             900             910
ACC GTC TGG GTC GCC GAC TCC TTC CAG GGC TTT CCC GAG CTG ACC GGG
THR VAL TRP VAL ALA ASP SER PHE GLN GLY PHE PRO GLU LEU THR GLY
      110                      115                     120


      920             930             940             950             960
TCC GAC CAC CCG CTG GAC GTC GAG ATC GAC CTC CAC CAG TAC AAC GAG
SER ASP HIS PRO LEU ASP VAL GLU ILE ASP LEU HIS GLN TYR ASN GLU
125                      130                     135                     140


      970             980             990             1000
GCC GTG GAC CTG CCC ACC AGC GAG GAG ACC GTG CGG GAG AAC TTC GCC
ALA VAL ASP LEU PRO THR SER GLU GLU THR VAL ARG GLU ASN PHE ALA
              145                     150                     155
```

```
    1010            1020              1030          1040            1050
CGG TAC GGG CTG CTC GAC GAC AAC GTC CGT TTC CTG GCG GGG TGG TTC
ARG TYR GLY LEU LEU ASP ASP ASN VAL ARG PHE LEU ALA GLY TRP PHE
              160                         165                     170


    1060            1070              1080          1090            1100
AAG GAC ACC ATG CCG GCT GCG CCC GTG AAG CAG CTC GCG GTG ATG CGC
LYS ASP THR MET PRO ALA ALA PRO VAL LYS GLN LEU ALA VAL MET ARG
              175                         180                     185


    1110            1120              1130          1140            1150
CTG GAC GGC GAC TCC TAC GGC GCC ACC ATG GAT GTG CTC GAC AGC CTG
LEU ASP GLY ASP SER TYR GLY ALA THR MET ASP VAL LEU ASP SER LEU
              190                         195                     200


    1160            1170              1180          1190            1200
TAC GAG CGG CTG TCG CCG GGC GGT TAC GTC ATC GTC GAC GAC TAC TGC
TYR GLU ARG LEU SER PRO GLY GLY TYR VAL ILE VAL ASP ASP TYR CYS
205                         210                   215                 220


    1210            1220              1230          1240
TC CCG GCC TGC CGC GAG CGG TGC ACG ACT TCC GCG ACC GGC TCG GCA
LE PRO ALA CYS ARG GLU ARG CYS THR THR SER ALA THR GLY SER ALA
              225                         230                     235


    1250            1260              1270          1280            1290
TCC GCG ACA CGA TCC ACC GGA TCG ACC GCC AGG GCG CTA TTG GCG GCA
SER ALA THR ARG SER THR GLY SER THR ALA ARG ALA LEU LEU ALA ALA
              240                         245                     250
```

63

```
        1300          1310         1320          1330          1340
CAG CGG CTG AGT CGT TCC GCC CGA GAG CCC GAC GAG AGC AGG AGA TAT
GLN ARG LEU SER ARG SER ALA ARG GLU PRO ASP GLU SER ARG ARG TYR
        255                        260                        265


        1350          1360          1370         1380          1390
GCG AGA CAC GAC GCG CCC GCT CGG CAT TGA GGG AGC GTG GGT GAT CCA
ALA ARG HIS ASP ALA PRO ALA ARG HIS
        270                        275


        1400          1410          1420          1430          1440
GCC GGA GAT CCA TCC GGA CCG GCG CGG CGA GTT CCA CGC GTG GTT CCA


        1450          1460          1470          1480
GAG CCA GCC GAG TTC CGG CGG CTG ACC GGT CAC TCC TTC TCC GTG CCG


   1490          1500          1510          1520          1530
CAG GTC GTC AAT ATC GCG TGT CCC GGA AAG GCG CCG CTG CGG CAT CCA


        1540          1550          1560          1570          1580
CTT CTG CCG AGG TGC CAC CGG GCC GAG GCC AAG TAC AGC GGC GTG TGT


        1590          1600          1610          1620          1630
GCA GGG CGC CGG TGT CGA GGT CGT CGT CGA CGC GCC GGT GTC GAG GTC


   1640
GTC GTC GAC-3'
```

dans laquelle A représente un résidu désoxyadényle, G représente un résidu désoxyguanyle, C représente un residu désoxycytidyle et T représente un résidu désoxythymidyle.

20. Séquence d'acides aminés codée par les nucléotides 541 à 1.371 du gène tylF défini à la revendication 19.

**Revendications pour les Etats contractants suivants : AT, ES**

1. Méthode pour accroître la capacité d'un micro-organisme de produire la tylosine ou un précurseur de la tylosine, qui comprend la culture d'un micro-organisme qui produit la tylosine ou un précurseur de la tylosine, ledit micro-organisme étant transformé avec un vecteur de clonage d'ADN ou une fraction de celui-ci qui contient un gène biosynthétique de la tylosine ou d'un précurseur de la tylosine comprenant

tylC disponible à partir du plasmide pHJL2804 (Figure 3), NRRL B-18044, ou à partir du plasmide pHJL311 (Figure 5), NRRL B-18046,

tylD disponible à partir du plasmide pHJL280 (Figure 2), NRRL B-18043, ou à partir du plasmide pHJL315 (Figure 6), NRRL B-18047,

tylE disponible à partir du plasmide pHJL280 (Figure 2), NRRL B-18043, ou à partir du plasmide pHJL315 (Figure 6), NRRL B-18047,

tylF disponible à partir du plasmide pHJL280 (Figure 2), NRRL B-18043, ou à partir du plasmide pHJL284 (Figure 3), NRRL B-18044, à partir du plasmide pHJL311 (Figure 5), NRRL B-18046, ou à partir du plasmide pHJL315 (Figure 6), NRRL B-18047,

tylH disponible à partir du plasmide pHJL280 (Figure 2), NRRL B-18043, à partir du plasmide pHJL311 (Figure 5), NRRL B-18046, ou à partir du plasmide pHJL315 (Figure 6), NRRL B-18047,

tylJ disponible à partir du plasmide pHJL280 (Figure 2), NRRL B-18043, à partir du plasmide pHJL284 (Figure 3), NRRL B-18044, à partir du plasmide pHJL311 (Figure 5), NRRL B-18046, ou à partir du plasmide pHJL315 (Figure 6), NRRL B-18047,

tylK disponible à partir du plasmide pHJL311 (Figure 5), NRRL B-18046,

tylL disponible à partir du plasmide pHJL309 (Figure 4), NRRL B-18045, ou

tylM disponible à partir du plasmide pHJL309 (Figure 4), NRRL B-18046,

dans des conditions appropriées à la croissance cellulaire, à l'expression du gène de la tylosine ou d'un précurseur de la tylosine et à la production de la tylosine ou d'un précurseur de la tylosine, pour autant que le procédé de culture fournit un accroissement de la capacité du microorganisme de produire la tylosine ou un précurseur de la tylosine.

2. Méthode selon la revendication 1, dans laquelle le micro-organisme est Streptomyces, Cephalosporium ou Penicillium.

3. Méthode selon la revendication 2, dans laquelle le micro-organisme est S. fradiae, S. rimosus et S. hygroscopicus.

4. Méthode selon la revendication 3, dans laquelle le gène biosynthétique de la tylosine est tylC, tylD, tylE, tylF, tylH, tylJ, tylK, tylL ou tylM.

5. Méthode selon l'une quelconque des revendications 1 à 4, dans laquelle le gène biosynthétique de la tylosine est tylF.

6. Méthode selon la revendication 1, dans laquelle le vecteur de clonage est le plasmide pHJL280, pHJL284, pHJL309, pHJL311 ou pHJL315.

7. Méthode selon la revendication 1 pour la production de Streptomyces fradiae GS15/pHJL280 en transformant S. fradiae GS15 (NRRL 18058) avec le plasmide pHJL280 (NRRL B-18043),
S. fradiae GS15/pHJL284 en transformant S. fradiae GS15 (NRRL 18058) avec le plasmide pHJL284 (NRRL B-18044),
S. fradiae GS15/pHJL311 en transformant S. fradiae GS15 (NRRL 18058) avec le plasmide pHJL311 (NRRL B-18046),
S. fradiae GS15/pHJL315 en transformant S. fradiae GS15 (NRRL 18058) avec le plasmide pHJL315 (NRRL B-18047),
S. fradiae GS28/pHJL280 en transformant S. fradiae GS28 (NRRL 18059) avec le plasmide pHJL280 (NRRL B-18043),
S. fradiae GS28/pHJL284 en transformant S. fradiae GS28 (NRRL 18059) avec le plasmide pHJL284 (NRRL B-18044),
S. fradiae GS28/pHJL311 en transformant S. fradiae GS28 (NRRL 18059) avec le plasmide pHJL311 (NRRL B-18046),
S. fradiae GS28/pHJL315 en transformant S. fradiae GS28 (NRRL 18059) avec le plasmide pHJL315 (NRRL B-18047),
S. fradiae GS16/pHJL280 en transformant S. fradiae GS16 avec le plasmide pHJL280 (NRRL B-18043),
S. fradiae GS16/pHJL315 en transformant S. fradiae GS16 avec le plasmide pHJL315 (NRRL B-18047),
S. fradiae GS48/pHJL280 en transformant S. fradiae GS48 avec le plasmide pHJL280 (NRRL B-18043),
S. fradiae GS48/pHJL315 en transformant S. fradiae GS48 avec le plasmide pHJL315 (NRRL B-18047),

S. fradiae GS52/pHJL284 en transformant S. fradiae GS52 (NRRL 18060) avec le plasmide pHJL284 (NRRL B-18044),

S. fradiae GS52/pHJL311 en transformant S. fradiae GS52 (NRRL 18060) avec le plasmide pHJL311 (NRRL B-18046),

S. fradiae GS76/pHJL280 en transformant S. fradiae GS76 avec le plasmide pHJL280 (NRRL B-18043), ou

S. fradiae GS76/pHJL315 en transformant S. fradiae GS76 avec le plasmide pHJL315 (NRRL B-18047).

8. Procédé pour la préparation de la tylosine, ou d'un sel pharmaceutiquement acceptable de celle-ci, qui comprend la culture d'un micro-organisme qui produit la tylosine ou un précurseur de la tylosine par une voie biosynthétique d'antibiotique, ledit micro-organisme étant transformé par un vecteur de clonage d'ADN, ou par une fraction de celui-ci, dans un milieu de culture contenant des sources assimilables de carbone, d'azote et de sels inorganiques dans des conditions de fermentation aérobies, caractérisé en ce que le vecteur de clonage d'ADN, ou une fraction de celui-ci, comprend un gène biosynthétique de la tylosine ou d'un précurseur de la tylosine, qui code pour l'expression d'une enzyme ou d'un produit de gène limitatif de la vitesse du gène biosynthétique de la tylosine ou d'un précurseur de la tylosine tel que défini à la revendication 1, qui code pour l'expression d'une enzyme ou d'un produit de gène limitatif de la vitesse de la voie biosynthétique de la tylosine ou d'un précurseur de la tylosine, ledit gène biosynthétique étant exprimé dans des conditions de fermentation permettant un accroissement de la capacité du micro-organisme de produire la tylosine ou un précurseur de la tylosine.

# FIG. I
# The Tylosin Biosynthetic Pathway

# FIG.2

## Restriction Site and Function Map of Plasmid pHJL280 (26.94 kb)

# FIG.3

## Restriction Site and Function Map of Plasmid pHJL284
## (26.94 kb)

# FIG.4

Restriction Site and Function Map of
Plasmid pHJL309
(28.24 kb)

# FIG.5

## Restriction Site and Function Map of Plasmid pHJL311 (30.84 kb)

EP 0 238 323 B1

# FIG.6

Restriction Site and Function Map of
Plasmid pHJL315
(38.83 kb)

# FIG.7
## Chromosomal Organization of the Tylosin Biosynthetic Genes